(19) Europäisches Patentamt
European Patent Office
Office européen des brevets

(11) EP 2 722 388 B1

(12) **EUROPEAN PATENT SPECIFICATION**

(45) Date of publication and mention of the grant of the patent:
**09.08.2017 Bulletin 2017/32**

(21) Application number: **12800770.5**

(22) Date of filing: **18.06.2012**

(51) Int Cl.:
***C12N 15/09*** *(2006.01)* ***C12Q 1/68*** *(2006.01)*

(86) International application number:
**PCT/JP2012/065529**

(87) International publication number:
**WO 2012/173274 (20.12.2012 Gazette 2012/51)**

# (54) NUCLEIC ACID PROBE FOR ASSAYING NUCLEIC ACIDS

NUKLEINSÄURESONDE ZUR ANALYSE VON NUKLEINSÄUREN

SONDE ACIDE NUCLÉIQUE POUR LE DOSAGE D'ACIDES NUCLÉIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2011 JP 2011134522**

(43) Date of publication of application:
**23.04.2014 Bulletin 2014/17**

(73) Proprietor: **Nippon Steel & Sumikin Eco-Tech Corporation**
**Chiyoda-ku**
**Tokyo 1010031 (JP)**

(72) Inventors:
- **ICHIKAWA Kohei**
  **Tokyo 101-0031 (JP)**
- **HANAWA Akiyoshi**
  **Tokyo 101-0031 (JP)**
- **KURATA Shinya**
  **Tokyo 101-0031 (JP)**

(74) Representative: **Blodig, Wolfgang**
**Wächtershäuser & Hartz**
**Patentanwaltspartnerschaft mbB**
**Weinstrasse 8**
**80333 München (DE)**

(56) References cited:
**JP-A- 2001 286 300**
**JP-A- 2008 182 974**
**JP-A- 2008 182 974**
**JP-A- 2010 088 304**
**US-A1- 2010 015 719**

- **FRIEDRICH ET AL: "DNA-probes for the highly sensitive identification of single nucleotide polymorphism using single-molecule spectroscopy", FEBS LETTERS, ELSEVIER, AMSTERDAM, NL, vol. 581, no. 8, 7 April 2007 (2007-04-07) , pages 1644-1648, XP022024558, ISSN: 0014-5793, DOI: 10.1016/J.FEBSLET.2007.03.031**
- **N. MARME: "Identification of single-point mutations in mycobacterial 16S rRNA sequences by confocal single-molecule fluorescence spectroscopy", NUCLEIC ACIDS RESEARCH, vol. 34, no. 13, 28 July 2006 (2006-07-28) , pages e90-e90, XP055134185, ISSN: 0305-1048, DOI: 10.1093/nar/gkl495**
- **"Custom Oligonucleotides", , 1 January 2010 (2010-01-01), XP055047426, Retrieved from the Internet: URL:http://www.anaspec.com/content/pdfs/c_literature188.pdf [retrieved on 2012-12-11]**
- **"ATTO 465", ATTO-TEC, 16 May 2011 (2011-05-16), XP055138653, Retrieved from the Internet: URL:http://www.atto-tec.com/fileadmin/user_upload/Katalog_Flyer_Support/ATTO_465.pdf [retrieved on 2014-09-08]**
- **"ATTO 655", ATTO-TEC, 16 May 2011 (2011-05-16), XP055138656, Retrieved from the Internet: URL:http://www.atto-tec.com/fileadmin/user_upload/Katalog_Flyer_Support/ATTO_655.pdf [retrieved on 2014-09-08]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 722 388 B1

- "ATTO 680", ATTO-TEC, 16 May 2011 (2011-05-16), XP055138659, Retrieved from the Internet: URL:http://www.atto-tec.com/fileadmin/user_upload/Katalog_Flyer_Support/ATTO_680.pdf [retrieved on 2014-09-08]
- "ATTO 700", ATTO-TEC, 16 May 2011 (2011-05-16), XP055138660, Retrieved from the Internet: URL:http://www.atto-tec.com/fileadmin/user_upload/Produktdatenblaetter/ATTO 700.pdf [retrieved on 2014-09-08]
- KURATA S ET AL: "Fluorescent quenching-based quantitative detection of specific DNA/RNA using a BODIPY((R)) FL-labeled probe or primer", NUCLEIC ACIDS RESEARCH, OXFORD UNIVERSITY PRESS, GB, vol. 29, no. 6, 15 March 2001 (2001-03-15) , page E34, XP002376104, ISSN: 0305-1048, DOI: 10.1093/NAR/29.6.E34
- TANI H ET AL: "Universal quenching probe system: flexible, specific, and cost-effective real-time polymerase chain reaction method", ANALYTICAL CHEMISTRY, AMERICAN CHEMICAL SOCIETY, US, vol. 81, no. 14, 15 July 2009 (2009-07-15) , pages 5678-5685, XP008136132, ISSN: 0003-2700, DOI: 10.1021/AC900414U [retrieved on 2009-06-16]
- 'Product Information: ATTO 465' 16 May 2011, XP055138653 Retrieved from the Internet: <URL:http://www.atto-tec.com/fileadmin/user_upload/Katalog_Flyer_Support/ATTO_465.pdf> [retrieved on 2012-07-05]
- 'Product Information: ATTO 655' 16 May 2011, XP055138656 Retrieved from the Internet: <URL:http://www.atto-tec.com/fileadmin/user_upload/Katalog_Flyer Support/ATTO 655.pdf> [retrieved on 2012-07-05]
- 'Product Information: ATTO 680' 16 May 2011, XP055138659 Retrieved from the Internet: <URL:http://www.atto-tec.com/fileadmin/user_upload/Katalog_Flyer_Support/ATTO_680.pdf> [retrieved on 2012-07-05]
- 'Product Information: ATTO 700' 16 May 2011, XP055138660 Retrieved from the Internet: <URL:http://www.atto-tec.com/fileadmin/user_upload/Katalog_Flyer_Support/ATTO_700.pdf> [retrieved on 2012-07-05]
- FRIEDRICH A. ET AL.: 'DNA-probes for the highly sensitive identification of single nucleotide polymorphism using single-molecule spectroscopy' FEBS LETT. vol. 581, no. 8, 2007, pages 1644 - 1648, XP022024558
- KUBOTA Y. ET AL.: 'Fluorescence decay studies of the DNA-3,6-diaminoacridine complexes' BIOPHYS. CHEM. vol. 19, no. 1, 1984, pages 25 - 37, XP055138664
- MARME N. ET AL.: 'Inter- and intramolecular fluorescence quenching of organic dyes by tryptophan' BIOCONJUG. CHEM. vol. 14, no. 6, 2003, pages 1133 - 1139, XP002332943
- TORIMURA M ET AL: "FLUORESCENCE-QUENCHING PHENOMENON BY PHOTOINDUCED ELECTRON TRANSFER BETWEEN A FLUORESCENT DYE AND A NUCLEOTIDE BASE", BUNSEKI-KAGAKU = ANALYTICAL CHEMISTRY, JAPAN SOCIETY FOR ANALYTICAL CHEMISTRY, TOKYO, JP, vol. 17, no. 1, 1 January 2001 (2001-01-01), pages 155-160, XP002953171, ISSN: 0910-6340, DOI: 10.2116/ANALSCI.17.155

## Description

### Technical Field

This invention relates to the field of genetic engineering, and more specifically, to a nucleic acid probe useful for analyzing nucleic acids.

### Background Art

In assaying target nucleic acids, nucleic acid probes are widely used. These nucleic acid probes have base sequences hybridizable specifically with the corresponding target nucleic acids, and are labeled with fluorescent substances. Among these nucleic acid probes, those attracting attention are fluorescence quenching probes labeled with fluorescence quenching dyes, the intensities of fluorescence from which are reduced upon coming close to guanine (see Non-patent Documents 1 to 3). Commercially-available fluorescence quenching probes include "QProbe" (registered trademark), "UniversalQProbe" (registered trademark: see Non-patent Document 2), and AB Probe (see Non-patent Document 3). By adding "QProbe" (registered trademark) to a solution to be measured and performing a measurement of fluorescence, SNP genotyping or quantification of a gene can be performed simply and conveniently. These fluorescence quenching probes have excellent advantages in that their structures are simple, no trial-and-error approach is needed for the designing of the probes, and highly-accurate measurement results can be obtained (see Patent Documents 1 to 5).

Those which are commercially used as fluorescence quenching dyes for labeling nucleic acid probes are four kinds of fluorescence quenching dyes, that is, BODIPY-FL, Pacific Blue, TAMRA and CR6G at present. These fluorescence quenching dyes are different from each other in fluorescence wavelength, so that by making use of these differences in fluorescence wavelength, plural kinds of target nucleic acids can be detected at the same time in a single reaction mixture.

For example, two kinds of target nucleic acids (target nucleic acids A and B) can be detected at the same time in a single reaction tube by adding a nucleic acid probe 1 for the target nucleic acid A, said nucleic acid probe 1 being labeled with TAMRA, and a nucleic acid probe 2 for the target nucleic acid B, said nucleic acid probe 2 being labeled with BODIPY-FL, to the same sample and measuring the intensity of florescence from TAMRA and the intensity of fluorescence from BODIPY-FL.

The prior art document US2010/015719 discloses Qprobes comprising fluorescent dyes whose fluorescence may be quenched by guanine in the complementary target polynucleotide.

### Prior Art Documents

### Non-patent Documents

Non-patent Document 1: Nucleic Acids Research, 29(6), e34 (2001)
Non-patent Document 2: Analytical Chemistry, 81(14), 5678-5685 (July 15, 2009)
Non-patent Document 3: Analytical Chemistry, 79(3), 974-979 (February 1, 2007)

### Patent Documents

Patent Document 1: JP-B-3437816
Patent Document 2: JP-B-3963422
Patent Document 3: JP-A-2008-182974
Patent Document 4: WO-A-2010/013317
Patent Document 5: WO-A-2010/013771

### Disclosure of the Invention

### Problem to Be Solved by the Invention

Keeping in step with the speed-up of gene analysis technologies in recent years, there are also outstanding

demands for a further speed-up of assay, concurrent detection of larger varieties of genes or target nucleic acids, and a reduction in assay cost in the field of assay of target nucleic acids that uses fluorescent quenching probes.

An object of the present invention is, therefore, to further speed up the assay of target nucleic acids, which uses fluorescence quenching probes, while suppressing increases in cost and labor.

**Means for Solving the Problem**

The above-described object can be achieved by the present invention as defined in the claims. Described specifically, the present invention provides a nucleic acid probe having a base sequence hybridizable with a target nucleic acid sequence in a target nucleic acid, wherein at least one terminal base is labeled with a fluorescence quenching dye, the base labeled with the fluorescence quenching dye is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is a fluorescence quenching dye selected from the group consisting of ATTO465, ATTO680 and ATTO700.

The present disclosure also provides a nucleic acid probe having a base sequence hybridizable with a target nucleic acid sequence in a target nucleic acid, wherein at least one terminal base is labeled with a fluorescence quenching dye, the base labeled with the fluorescence quenching dye is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and fluorescence at a predetermined wavelength in a wavelength range of 600 nm to 750 nm is lowered to 40% or less in intensity when hybridized with the target nucleic acid sequence compared with when not hybridized with the target nucleic acid sequence.

**Advantageous Effects of the Invention**

According to the present invention, there are provided nucleic acid probes, which in the detection of target nucleic acids with fluorescent quenching probes, can assay a greater variety of target nucleic acids at the same time while suppressing increases in assay labor and cost, and also a method for assaying target nucleic acids by using the nucleic acid probes.

**Brief Description of the Drawings**

FIG. 1 is a schematic view illustrating one example of a nucleic acid probe according to the present invention.
FIG. 2 is a graph showing the intensity of fluorescence from a fluorescence quenching probe labeled with ATT0655.
FIG. 3 is a graph showing the intensity of fluorescence from a fluorescence quenching probe labeled with ATTO680.
FIG. 4 is a graph showing the intensity of fluorescence from a fluorescence quenching probe labeled with ATTO700.
FIG. 5 is a schematic view illustrating of one example of a nucleic acid probe set according to the present invention.
FIG. 6 is a schematic view illustrating of another example of the nucleic acid probe set according to the present invention.
FIG. 7 is a graph showing the temperature dependence of fluorescence intensity.
FIG. 8 is a graph showing the temperature dependence of fluorescence quenching efficiency.
FIG. 9 is a graph showing the first negative derivatives of melting curves.
FIG. 10 is a graph showing the results of a real-time PCR assay.
FIG. 11 is a graph showing the results of SNP genotyping.
FIG. 12 is a graph showing the temperature dependence of fluorescence intensity.
FIG. 13 is a graph showing the temperature dependence of fluorescence quenching efficiency.
FIG. 14 is a graph showing the results of SNP genotyping.

**Modes for Carrying out the Invention**

Modes for carrying out the present invention will next be described in detail. It is to be noted that the term "nucleotide" as used alone in the present invention means a monomer that makes up a polynucleotide and shall not be limited to a deoxyribonucleotide as a constituent unit of DNA or a ribonucleotide as a constituent unit of RNA but can

be an artificially-synthesized monomer such as LNA (Locked Nucleic Acid), peptide nucleic acid (PNA), ENA (2'-0,4'-C-Ethylene-bridged Nucleic Acids), 2',4'-$BNA^{NC}$ or 2',4'-$BNA^{COC}$.

The term "oligonucleotide" as used herein means an oligomer formed from a nucleotide monomer, and this oligomer may be formed solely of deoxyribonucleotide, ribonucleotide, LNA or PNA units or may be a chimeric molecule of such units.

Further, the terms "nucleic acid" and "polynucleotide" as used herein mean polymers formed from the above-described nucleotides.

The term "target nucleic acid" as used herein means a nucleic acid which is a subject of quantification, analysis or the like, and shall embrace such a case that the nucleic acid is a part of one of various nucleic acids or genes.

The terms "target nucleic acid sequence" as used herein means a base sequence region in a target nucleic acid, said base sequence region being to be paired with a nucleic acid probe according to the present invention upon hybridization of the nucleic acid probe. When the target nucleic acid is a nucleic acid having a long base length such as a gene or plasmid, the base sequence region is used to identify a base sequence region that is to be paired with the nucleic acid probe according to the present invention.

The nucleic acid probe according to the present invention is a nucleic acid probe formed of a single-stranded oligonucleotide, and is required to have a base sequence hybridizable with the target nucleic acid sequence. As an example of the base sequence hybridizable with the target nucleic acid sequence, a base sequence complementary to the target nucleic acid sequence can be mentioned. When the base length of the target nucleic acid is short, the target nucleic acid may be the target nucleic acid sequence. When the target nucleic acid is a nucleic acid having a long base length such as a gene or plasmid, the base sequence of the nucleic acid probe according to the present invention should be a base sequence hybridizable with a target nucleic acid sequence as a part of the target nucleic acid. The base sequence of the nucleic acid probe according to the present invention is not required to be fully complementary to the target nucleic acid sequence, but may have a complementation of preferably 85% or greater, more preferably 90% or greater, still more preferably 95% or greater.

No particular limitation is imposed on the base length of the nucleic acid probe according to the present invention insofar as the nucleic acid probe is hybridizable with the target nucleic acid or target nucleic acid sequence. In the case of a DNA probe formed solely of a deoxyribonucleotide as a monomer, however, a length of 4 bases or less may not be preferred in that it may lead to a lower hybridization efficiency, and a length of 51 bases or more may not be preferred either in that it tends to form non-specific hybrids. When the nucleic acid probe according to the present invention is a DNA probe, its base length may, therefore, be preferably 5 to 50 bases long, more preferably 10 to 35 bases long, especially preferably 10 to 20 bases long.

The nucleic acid probe according to the present invention may be a DNA probe formed solely of a deoxyribonucleotide as a monomer, or instead of such a deoxyribonucleotide, may contain LNA or BNA having stronger base-to-base interaction. Compared with the nucleic acid probe formed solely of the deoxyribonucleotide, the use of LNA or BNA can control Tm (melting temperature) at a similar level with a base length shorter than that of the DNA probe.

The nucleic acid probe according to the present invention is labeled with the below-mentioned fluorescent quenching substance at least one terminal base thereof. The base labeled with the fluorescence quenching dye is required to be cytosine.

The position labeled with the fluorescence quenching dye may be any one of a sugar site, a phosphate site and a base site of the nucleotide. At the sugar site, the labeled position is an OH group on the 3'- or 2'-C atom at the 3' -end or an OH group on the 5' -C atom as obtained by dephosphorylating the 5' -end. The labeling may be conducted after substituting the phosphate site with a sulfonate group or sulfonyl group.

When desired to label a 5'-terminal nucleotide with a fluorescence quenching dye, it is necessary to first introduce, for example, $-(CH_2)_n-SH$ as a linker to the 5'-terminal phosphate group in a manner known *per se* in the art. As such a linker, a commercial linker can be used (for example, Midland Certified Reagent Company, U.S.A). In this case, n may stand for 3 to 8, with 6 being preferred. By coupling a fluorescence quenching dye, which has reactivity to an SH group, or a derivative thereof to this linker, an oligonucleotide labeled with the fluorescence quenching dye can be obtained. The fluorescently-labeled oligonucleotide can be purified by reverse phase chromatography or the like to provide the nucleic acid probe according to the present invention.

It is to be noted that, when a nucleic acid probe labeled with a fluorescence quenching dye at the 5'-end of an oligonucleotide is used along with a polymerase, a measure such as phosphorylation of the 3'-end should be taken to prevent any extension from the 3'-end.

As an alternative, the oligonucleotide can also be labeled with the fluorescence quenching dye at the 3'-terminal nucleotide unit thereof. In this case, it is necessary to introduce, for example, $-(CH_2)_n-NH_2$ as a linker to the OH group on the 3'-C atom of ribose or deoxyribose. As such a linker, a commercial linker can also be used (for example, Midland Certified Reagent Company, U.S.A). As an alternative method, it is also possible to introduce a phosphate group to the OH group on the 3'-C atom of ribose or deoxyribose and then to introduce, for example, $-(CH_2)_n-SH$ as a linker to the OH group in the phosphate group. In this case, n may stand for 3 to 8, with 4 to 7 being preferred.

By coupling a fluorescence quenching dye, which has reactivity to an amino group or SH group, or a derivative thereof to the above-described linker, an oligonucleotide labeled with the fluorescence quenching dye can be synthesized. The oligonucleotide can be purified by reverse phase chromatography or the like to provide the nucleic acid probe according to the present invention. When desired to introduce $-(CH_2)_n-NH_2$ as a linker, it is convenient to use a kit reagent (for example, Uni-link aminomodifier, Clonetech Laboratories, Inc.). The fluorescence quenching dye can then be coupled to the oligonucleotide in a manner known *per se* in the art.

The assay of the target nucleic acid makes use of a phenomenon that upon hybridization of the nucleic acid probe according to the present invention with the target nucleic acid sequence in the target nucleic acid, the fluorescence quenching dye in the nucleic acid probe and the guanine base in the target nucleic acid sequence come to close to each other, and as a result, the fluorescence emission of the fluorescence quenching dye quenches than that in the normal case. It is, therefore, necessary to design the nucleic acid probe according to the present invention such that the nucleic acid probe has a base sequence hybridizable with the target nucleic acid sequence, and upon hybridization with the target nucleic acid sequence, the fluorescence quenching dye in the nucleic acid probe and the guanine base in the target nucleic acid sequence come close to each other. Describing with reference to FIG. 1, it is necessary to design the base sequence of a nucleic acid probe 5 according to the present invention such that upon hybridization of the nucleic acid probe 5 with a target nucleic acid sequence 11 in a target nucleic acid 10, a base (which is indicated by "1" in FIG. 1, and will hereinafter be abbreviated as "the base 1") in the target nucleic acid sequence 11, said base 1 being to be located at a position where it is to be paired with a cytosine base labeled with a fluorescence quenching dye 4 in the nucleic acid probe 5 is a guanine base, or at least one guanine base exists in a range of five bases (which are the bases indicated by "1", "2" and "3" in FIG. 1) centering at the base (the base 1) in the target nucleic acid sequence 11, said base 1 being to be paired with the cytosine base. Insofar as a guanine base exists in the range of the above-described five bases, the hybridization of the nucleic acid probe 5 according to the present invention to the target nucleic acid sequence 11 leads to a signification reduction in the intensity of fluorescence from the fluorescence quenching dye 4, and this reduction can be used for the assay of the target nucleic acid 10.

The extent of fluorescence quenching upon hybridization of a fluorescence quenching probe with a target nucleic acid sequence in a target nucleic acid can be evaluated as a fluorescence quenching efficiency determined according to the following equation (1):

$$\text{Fluorescence quenching efficiency (\%)} = (F_1-F_2)/F_1\times 100 \quad (1)$$

where,

- $F_1$ is a fluorescence intensity under conditions that the target nucleic acid sequence does not exist or under conditions that the target nucleic acid sequence and the nucleic acid probe according to the present invention are not hybridizable with each other, and
- $F_2$ is a fluorescence intensity under conditions that the target nucleic acid sequence and the nucleic acid probe according to the present invention exist together or under conditions that the target nucleic acid sequence and the nucleic acid probe according to the present invention are hybridizable with each other.

The fluorescence quenching efficiency required upon assaying a target nucleic acid also depends on the measurement wavelength and absolute fluorescence intensity, and cannot therefore be specified. However, a fluorescence quenching efficiency of 40% or larger may be preferred because it can clearly identify fluorescence quenching. The fluorescence quenching efficiency may be more preferably 50% or larger, still more preferably 60% or larger.

The nucleic acid probe according to the present invention uses a compound selected from the group consisting of ATTO465, ATTO680 and ATTO700. ATTO465, ATTO655, ATT0680 and ATTO700 are all available from ATTO-TEC GmbH. Their catalog numbers are as follows: AD 465-31 for ATTO465, AD 655-31 and AD 655-35 for ATTO655, AD 680-31 and AD 680-35 for ATTO680, and AD 700-31 and AD 700-35 for ATTO700.

ATTO465 is a derivative of acriflavine, and has a structure represented by the following chemical formula (1):

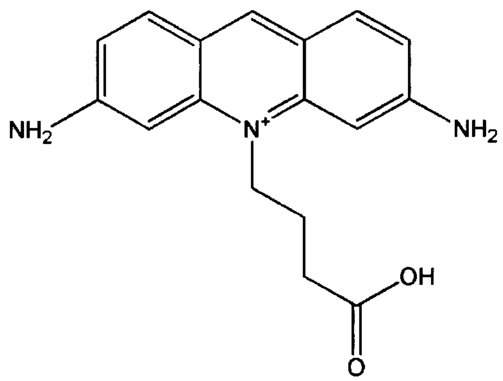

An⁻ (1)

The systematic name of ATTO655 is 1-(3-carboxypropyl)-11-ethyl-2,2-dimethyl-4-(sulfon atomethyl)-3,4,8,9,10,11-hexahydro-2H-13-oxa-1,6,11 -triazapentacene-1-ium, which has the following chemical formula (2):

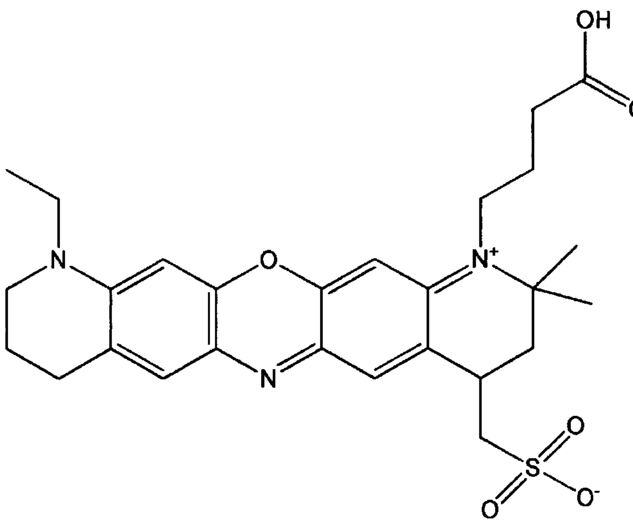

(2)

In a free state, the nucleic acid probe according to the present invention as labeled with ATT0465 exhibits intense fluorescence in a wavelength range of 490 nm to 550 nm, and its fluorescence intensity reaches the maximum around 508 nm. The present inventors found that upon hybridization of a nucleic acid probe, which is labeled with ATT0465 at a terminal cytosine base thereof, with a target nucleic acid sequence in a target nucleic acid, the intensity of fluorescence from the nucleic acid probe becomes considerably weaker compared with that in a free state when a base in the target nucleic acid sequence, said base being to be paired with the cytosine base, is a guanine base, or at least one guanine base exists in a range of five bases centering at the base in the target nucleic acid sequence, said base being to be paired with the cytosine base. As the extent of this fluorescence quenching is pronounced around 508 nm, it is preferred to perform a measurement of fluorescence around 508 nm when the nucleic acid probe labeled with ATT0465 is used for the assay of the target nucleic acid. As an excitation wavelength, it is preferred to use a wavelength around 453 nm.

In a free state, the nucleic acid probe according to the present disclosure as labeled with ATTO655 exhibits intense fluorescence in a wavelength range of 660 nm to 720 nm as shown by cross marks (X) in FIG. 2, and its fluorescence intensity reaches the maximum around 684 nm. As disclosed herein, upon hybridization of a nucleic acid probe, which is labeled with ATT0655 at a terminal cytosine base thereof, with a target nucleic acid sequence in a target nucleic acid, fluorescence is pronouncedly quenched, compared with that before the hybridization, in a wavelength range of 680 nm to 800 nm as shown by circles (O) in FIG. 2 when a base in the target nucleic acid sequence, said base being to be paired with the cytosine base, is a guanine base, or at least one guanine base exists in a range of five bases centering at the base in the target nucleic acid sequence, said base being to be paired with the cytosine base. When the target nucleic acid is assayed using the nucleic acid probe labeled with ATTO655, it is preferred to perform a measurement of fluorescence around 684 nm where the fluorescence quenching efficiency reaches the maximum. As an excitation wavelength, it is preferred to use a wavelength around 663 nm.

In a free state, the nucleic acid probe according to the present invention as labeled with ATT0680 exhibits

intense fluorescence in a wavelength range of 690 nm to 760 nm as shown by cross marks (X) in FIG. 3, and its fluorescence intensity reaches the maximum around 712 nm.

The present inventors found that upon hybridization of a nucleic acid probe, which is labeled with ATTO680 at a terminal cytosine base thereof, with a target nucleic acid sequence in a target nucleic acid, pronounced fluorescence quenching is exhibited, compared with that before the hybridization, in a wavelength range of 690 nm to 760 nm as shown by circles (O) in FIG. 3 when a base in the target nucleic acid sequence, said base being to be paired with the cytosine base, is a guanine base, or at least one guanine base exists in a range of five bases centering at the base in the target nucleic acid sequence, said base being to be paired with the cytosine base. When the target nucleic acid is assayed using the nucleic acid probe labeled with ATT0680, it is preferred to perform a measurement of fluorescence around 700 nm where the fluorescence quenching efficiency reaches the maximum. As an excitation wavelength, it is preferred to use a wavelength around 680 nm.

In a free state, the nucleic acid probe according to the present invention as labeled with ATTO700 exhibits intense fluorescence in a wavelength range of 690 nm to 760 nm as shown by cross marks (X) in FIG. 4, and its fluorescence intensity reaches the maximum around 702 nm.

The present inventors found that upon hybridization of a nucleic acid probe, which is labeled with ATTO700 at a terminal cytosine base thereof, with a target nucleic acid sequence in a target nucleic acid, pronounced fluorescence quenching is exhibited, compared with that before the hybridization, in a wavelength range of 720 nm to 760 nm as shown by circles (O) in FIG. 4 when a base in the target nucleic acid sequence, said base being to be paired with the cytosine base, is a guanine base, or at least one guanine base exists in a range of five bases centering at the base in the target nucleic acid sequence, said base being to be paired with the cytosine base. When the target nucleic acid is assayed using the nucleic acid probe labeled with ATT0700, it is preferred to perform a measurement of fluorescence around 720 nm where the fluorescence quenching efficiency is high and the fluorescence intensity in the free state is also high. As an excitation wavelength, it is preferred to use a wavelength around 700 nm.

Those which have heretofore been commercially used as fluorescence quenching dyes in fluorescence quenching nucleic acid probes are four kinds of fluorescence quenching dyes, that is, 4,4-difluoro-5,7-dimethyl-4-bora-3a,4a-diaza-s-inda cene-3-propionic acid (available under the trade name of "BODIPY-FL" from Invitrogen Corp.), 2-oxo-6,8-difluoro-7-hydroxy-2H-1-benzopyran-3-carb oxylic acid (available under the trade name of "Pacific Blue" from Invitrogen Corp.), TAMRA (carboxytetramethylrhodamine), and CR6G (carboxyrhodamine 6G).

When fluorescence quenching by a proximal guanine base is used, the fluorescence measuring wavelength is around 455 nm for Pacific Blue, around 513 nm for BODIPY-FL, around 555 nm for CR6G, or around 580 nm for TAMRA. By labeling nucleic acid probes of different sequences with these four kinds of fluorescence quenching dyes, respectively, it was possible to detect up to four kinds of target nucleic acid sequences at the same time in a single reaction mixture. In other words, the fluorescence quenching dyes, which have been commercially used as labels in fluorescence quenching probes, are only four fluorescence quenching dyes. Accordingly, the number of target nucleic acid sequences which can be detected at the same time in a single system has been limited to four even at a maximum.

As described above, the fluorescence measuring wavelengths for conventionally-used fluorescence quenching probes have been limited to a wavelength range of 440 to 590 nm. The wavelength range which can be observed by a conventional florescence measuring system is 200 nm to 1,000 nm in general. Using a fluorescence quenching probe that the intensity of fluorescence at a predetermined wavelength, for example, in a wavelength range of 600 nm to 750 nm significantly decreases (for example, to 40% or less) by a proximal guanine base, the number of target nucleic acid sequences detectable at the same time in a single system by using the conventional fluorescence measuring system increases accordingly.

The fluorescence quenching measurement wavelengths for the above-described nucleic acid probes according to the present disclosure are apart from each other (for example, ATTO465: 508 nm, ATTO655: 684 nm, ATTO680: 700 nm, ATTO700: 720 nm), so that fluorescence derived from the respective probes can be distinguished even when the four kinds of probes are used in combination in a single sample. Further, the fluorescence quenching measurement wavelengths for the nucleic acid probes according to the present disclosure are also different from those for the above-described four kinds of conventional fluorescence quenching probes, and fall within the observation wavelength range of a general fluorescence measuring system. Therefore, the nucleic acid probes according to the present disclosure can be used instead of the conventional fluorescence quenching probes. The nucleic acid probes according to the present disclosure can also be used by adding them together with the conventional fluorescence quenching probes to a measurement system, thereby making it possible to detect up to eight kinds of target nucleic acid sequences at the same time in a single sample without an increase in measurement cost and hence to achieve a further speed-up and cost reduction in the assay of target nucleic acids.

The nucleic acid probes according to the present invention can also be used, in place of the conventional fluorescence quenching probes, in the analysis of a nucleic acid for a base sequence polymorphism.

In another aspect of the nucleic acid probe according to the present disclosure, there is also provided a nucleic acid probe having a base sequence hybridizable with a target nucleic acid sequence in a target nucleic acid, wherein

at least one terminal base is labeled with a fluorescence quenching dye, the base labeled with the fluorescence quenching dye is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and fluorescence at a predetermined wavelength in the wavelength range of 600 nm to 750 nm is lowered to 40% or less in intensity when hybridized with the target nucleic acid sequence compared with when not hybridized with the target nucleic acid sequence.

As described above, the measurement wavelengths for fluorescence quenching by proximation between guanines and the fluorescence quenching dyes have heretofore been limited to the wavelength range of 440 to 590 nm. The use of the fluorescence quenching probe according to the present disclosure that the intensity of fluorescence at the predetermined wavelength in the wavelength range of 600 nm to 750 nm is lowered to 40% or less by proximal guanine can increase the kinds of target nucleic acid sequences detectable at the same time in a single system and can achieve a further speed-up and cost reduction in the assay of target nucleic acids.

As the fluorescence quenching dye for use in the above-described fluorescence quenching probe, examples include the fluorescent substances described in US-A-2006/0179585. As specific examples of preferred fluorescence quenching dyes, ATTO655, ATTO680 and ATT0700 can be mentioned.

Further, upon designing the base sequence of the nucleic acid probe according to the present invention, it is preferred to set the guanine content as low as possible in a range not higher than 25%, to set the GC content at 70% or lower, and to select a base other than guanine as the base adjacent the base labeled with the fluorescence quenching dye.

As a further aspect of the present invention, there is also provided a target nucleic acid sequence assaying kit comprising a first nucleic acid probe having a base sequence hybridizable with a first target nucleic acid sequence, wherein at least one terminal base is labeled with a fluorescence quenching dye, the base labeled with the fluorescence quenching dye is cytosine, the base sequence is designed such that upon hybridization of the nucleic acid probe with the first target nucleic acid sequence, a base in the first target nucleic acid sequence, said base being to be paired with the cytosine base, is a guanine base, or at least one guanine base exists in a range of five bases centering at the base in the first target nucleic acid sequence, said base being to be paired with the cytosine base, and the fluorescence quenching dye is a fluorescence quenching dye selected from the group consisting of BODIPY-FL, Pacific Blue, TAMRA and CR6G; and

a second nucleic acid probe having a base sequence hybridizable with a second target nucleic acid sequence, wherein at least one terminal base is labeled with a fluorescence quenching dye, the base labeled with the fluorescence quenching dye is cytosine, the base sequence is designed such that upon hybridization of the nucleic acid probe with the second target nucleic acid sequence, a base in the second target nucleic acid sequence, said base being to be paired with the cytosine base, is a guanine base, or at least one guanine base exists in a range of five bases centering at the base in the second target nucleic acid sequence, said base being to be paired with the cytosine base, and the fluorescence quenching dye is a fluorescence quenching dye selected from the group consisting of ATTO465, ATTO680 and ATTO700.

The target nucleic acid sequence assaying kit uses, in addition to the first nucleic acid probe as a conventional fluorescence quenching probe, the second nucleic acid probe different in the measurement wavelength for fluorescence quenching from the first nucleic acid probe, and uses a wavelength range, which is different from the measurement wavelength for the conventional fluorescence quenching probe, for the assay of the second target nucleic acid sequence. The first target nucleic acid sequence and second target nucleic acid sequence may be on the same target nucleic acid (for example, gene), or may be sequences in different target nucleic acids (genes). By setting the first target nucleic acid sequence and second target nucleic acid sequence on the same gene, it is possible to assay, for example, mutations at two locations on a single gene at the same time. By setting the first target nucleic acid sequence and second target nucleic acid sequence on different genes, on the other hand, it is possible to assay two genes in a single sample at the same time.

As another preferred embodiment of the above-described target nucleic acid sequence assaying kit, there can be mentioned a target nucleic acid sequence assaying kit comprising five or more nucleic acid probes selected from the group consisting of:

(1) a first nucleic acid probe having a base sequence hybridizable with a first target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is BODIPY-FL;

(2) a second nucleic acid probe having a base sequence hybridizable with a second target nucleic acid sequence, wherein at least one terminal base is labeled with a fluorescence quenching dye, the labeled base is cytosine, the

base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is Pacific Blue;

(3) a third nucleic acid probe having a base sequence hybridizable with a third target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is TAMRA;

(4) a fourth nucleic acid probe having a base sequence hybridizable with a fourth target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is CR6G;

(5) a fifth nucleic acid probe having a base sequence hybridizable with a fifth target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is ATTO465;

(6) a sixth nucleic acid probe having a base sequence hybridizable with a sixth target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is ATT0655;

(7) a seventh nucleic acid probe having a base sequence hybridizable with a seventh target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is ATTO680; and

(8) a eighth nucleic acid probe having a base sequence hybridizable with an eighth target nucleic acid sequence, wherein at least one terminal base is labeled with a fluorescence quenching dye, the labeled base is cytosine, the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and the fluorescence quenching dye is ATTO700.

As the fluorescence quenching dyes which have heretofore been commercially used in fluorescence quenching probes are only four kinds of fluorescence quenching dyes, the upper limit of the kinds of target nucleic acid sequences which can be assayed at the same time in a single assay system (sample) has been 4. However, the use of the above-described target nucleic acid sequence assaying kit according to the present invention can assay, in a single reaction mixture, five kinds of target nucleic acid sequences when the kit contains five kinds of nucleic acid probes or eight kinds of target nucleic acid sequences when the kit contains eight kinds of nucleic acid probes, thereby making it possible to achieve a further speed-up and cost reduction in the assay of many kinds of target nucleic acids or target nucleic acid sequences.

According to a still further aspect of the present invention, there is also provided a method for detecting target nucleic acid sequences in a sample, comprising the following steps: adding a first nucleic acid probe and a second nucleic acid probe to the sample, measuring fluorescence derived from the first nucleic acid probe and fluorescence derived from the second nucleic acid probe while maintaining the sample under conditions where the nucleic acid probes and the first and second ones of the target nucleic acid sequences are hybridizable, respectively, and measuring fluorescence derived from the first nucleic acid probe and fluorescence derived from the second nucleic acid probe while maintaining the sample under conditions where the nucleic acid probes and the first and second ones of the target

nucleic acid sequences are not hybridizable, respectively, wherein:

the first nucleic acid probe has a base sequence hybridizable with the first target nucleic acid sequence, at least one terminal base is labeled with a fluorescence quenching dye, the base labeled with the fluorescence quenching dye is cytosine, the base sequence is designed such that upon hybridization of the first nucleic acid probe with the first target nucleic acid sequence, a base in the first target nucleic acid sequence, said base being to be paired with the cytosine base, is a guanine base, or at least one guanine base exists in a range of five bases centering at the base in the first target nucleic acid sequence, said base being to be paired with the cytosine base, and the fluorescence quenching dye is a fluorescence quenching dye selected from the group consisting of BODIPY-FL, Pacific Blue, TAMRA and CR6G; and the second nucleic acid probe has a base sequence hybridizable with the second target nucleic acid sequence, at least one terminal base is labeled with a fluorescence quenching dye, the base labeled with the fluorescence quenching dye is cytosine, the base sequence is designed such that upon hybridization of the second nucleic acid probe with the second target nucleic acid sequence, a base in the second target nucleic acid sequence, said base being to be paired with the cytosine base, is a guanine base, or at least one guanine base exists in a range of five bases centering at the base in the second target nucleic acid sequence, said base being to be paired with the cytosine base, and the fluorescence quenching dye is a fluorescence quenching dye selected from the group consisting of ATTO465, ATTO680 and ATTO700.

The above-described detection method according to the present invention for the target nucleic acid sequences uses, in addition to the first nucleic acid probe as a conventional fluorescence quenching probe, the second nucleic acid probe different in fluorescence quenching measurement wavelength from the first nucleic acid probe, and uses a wavelength range, which is different from the measurement wavelength for the conventional fluorescence quenching probe, for the assay of the second target nucleic acid sequence. Similar to the above-described target nucleic acid sequence assaying kit, the first target nucleic acid sequence and second target nucleic acid sequence may be on the same target nucleic acid (for example, gene), or may be sequences in different target nucleic acids (genes).

Third and fourth nucleic acid probes, which are different in base sequence from the first and second nucleic acid probes, may be used further, thereby making it possible to detect still more target nucleic acid sequences in the single sample.

According to an even further aspect of the present invention, there is also provided a nucleic acid probe set comprising a binding probe and a fluorescent probe, wherein:

the binding probe comprises an oligonucleotide, and includes a target nucleic acid binding region hybridizable with a target nucleic acid sequence and a fluorescent probe binding region hybridizable with the fluorescent probe,

the target nucleic acid binding region has a base sequence designed such that upon hybridization with the target nucleic acid sequence, a base in the target nucleic acid sequence, said base being to be paired with a base on a side of the fluorescent probe binding region out of terminal bases in the target nucleic acid binding region, is a guanine base, or at least one guanine base exists in a range of five bases centering at the base in the target nucleic acid sequence, said base being to be paired with the terminal base, and

the fluorescent probe comprises an oligonucleotide, and is labeled with a fluorescence quenching dye at a 3'-terminal base thereof when the fluorescent probe binding region is located on a side of a 3' -end of the binding probe or is labeled with the fluorescence quenching dye at a 5'-terminal base thereof when the fluorescent probe binding region is located on a side of a 5'-end of the binding probe, and the fluorescence quenching dye is selected from the group consisting of ATTO465, ATTO680 and ATTO700.

The above-described nucleic acid probe set according to the present invention is a universal fluorescence quenching probe similar to those described in Non-patent Document 2 cited above. A schematic diagram of one example of the nucleic acid probe set according to the present invention is illustrated in FIG. 5. The nucleic acid probe set illustrated in FIG. 5 is labeled with a fluorescence quenching dye 4 at the 3'-terminal base of a fluorescent probe 6 because a fluorescent probe binding region 7 is located on the side of the 3'-end of a binding probe 9. The fluorescent probe 6 that makes up the nucleic acid probe set according to the present invention is simply required to have a base sequence hybridizable with the fluorescent probe binding region 7, and the same fluorescent probe 6 can be used irrespective of which one of different target nucleic acid 10 and target nucleic acid sequence 11 is to be assayed. Upon performing an analysis of the target nucleic acid 10 or target nucleic acid sequence 11 by using the nucleic acid probe set according to the present invention, it is thus unnecessary to prepare the fluorescent probe 6, which contains the fluorescence quenching dye 4 and is costly, separately for each of the analytes. Cost for such an analysis can be suppressed accordingly.

In the nucleic acid probe set according to the present invention, a compound selected from the group consisting of ATTO465, ATTO680 and ATTO700 is used as the fluorescence quenching dye 4. When ATTO465, ATTO680 and

ATTO700 each come close to guanine, the fluorescence quenches compared with that in a free state. Based on the fluorescence quenching action of the fluorescence quenching dye 4, the nucleic acid probe set according to the present invention can, therefore, be suitably used for the analysis of the target nucleic acid sequence 11 by designing the base sequence of the target nucleic acid binding region 8 such that the target nucleic acid binding region 8 is hybridizable with the target nucleic acid sequence 11 and a base in the target nucleic acid sequence 11, said base being to be paired with a base (indicated by "12" in FIG. 5) on the side of the fluorescent probe binding region 7 out of the terminal bases of the target nucleic acid binding region 8, is a guanine base, or at least one guanine base exists in a range of five bases (the bases indicated by "1", "2" or "3" in FIG. 5) centering at the base 1, and further by labeling the 3' -terminal base of the fluorescent probe 6 with the fluorescence quenching dye 4 when the fluorescent probe binding region 7 is located on the side of the 3'-end of the binding probe 9 or labeling the 5' -terminal base of the fluorescent probe 6 with the fluorescence quenching dye 4 when the fluorescent probe binding region 7 is located on the side of the 5'-end of the binding probe 9.

The nucleic acid probe set according to the present invention includes two configurations, one being a case where the target nucleic acid binding region 8 is located on the side of the 5'-end of the binding probe 9, and the other a case where the target nucleic acid binding region 8 is located on the side of the 3' -end of the binding probe 9. However, the case where the target nucleic acid binding region 8 is placed on the side of the 5'-end of the binding probe 9 as illustrated in FIG. 5 exhibits a higher fluorescence quenching efficiency than the case where the target nucleic acid binding region 8 is placed on the side of the 3'-end of the binding probe 9, and is therefore preferred.

In the fluorescent probe 6, the terminal base labeled with the fluorescence quenching dye 4 may preferably be either adenine or thymine.

Assuming that the melting point of the complex of the fluorescent probe 6 and binding probe 9, which make up the nucleic acid probe set according to the present invention, is "Tm2" and the melting point of the complex of the hybridized complex of the fluorescent probe 6 and binding probe 9 and the target nucleic acid sequence is "Tm1", Tm2 may preferably be higher than Tm1, with a Tm2-Tm1 value of 5°C or greater being more preferred. Further, Tm2 may preferably be 95°C or higher.

According to a yet further aspect of the present invention, there is also provided a nucleic acid probe set comprising a binding probe, a first fluorescent probe and a second fluorescent probe, wherein:

the binding probe comprises an oligonucleotide, and has, from a side of a 5'-end thereof, a first fluorescent probe binding region hybridizable with the first fluorescent probe, a target nucleic acid binding region hybridizable with a target nucleic acid sequence, and a second fluorescent probe binding region hybridizable with the second fluorescent probe,

the target nucleic acid binding region has a base sequence designed such that upon hybridization with the target nucleic acid sequence, a base in the target nucleic acid sequence, said base being to be paired with a 5'-side terminal base in the target nucleic acid binding region, is a guanine base or at least one guanine base exists in a range of five bases centering at the base in the target nucleic acid sequence, said base being to be paired with the terminal base, and a base in the target nucleic acid sequence, said base being to be paired with a 3'-side terminal base in the target nucleic acid binding region, is a guanine base or at least one guanine base exists in a range of five bases centering at the base in the target nucleic acid sequence, said base being to be paired with the terminal base,

the first fluorescent probe comprises an oligonucleotide, and is labeled with a first fluorescence quenching dye at a 5'-terminal base thereof,

the second fluorescent probe comprises an oligonucleotide, and is labeled with a second fluorescence quenching dye at a 3'-terminal base thereof,

the first fluorescence quenching dye is different from the second fluorescence quenching dye,

one of the first fluorescence quenching dye and second fluorescence quenching dye is a fluorescence quenching dye selected from the group consisting of ATTO465, ATT0655, ATTO680 and ATT0700, and

the other fluorescence quenching dye is a fluorescence quenching dye selected from the group consisting of BODIPY-FL, Pacific Blue, TAMRA, CR6G, ATTO465, ATTO680 and ATTO700.

A schematic diagram of another example of the above-described probe set is illustrated in FIG. 6. The nucleic acid probe set illustrated in FIG. 6 can be used as a replacement for an AB probe which has conventionally been used in the ABC-PCR (Alternately Binding Probe Competitive PCR) method (see Non-patent Document 3). BODIPY-FL, Pacific Blue, TAMRA, CR6G, ATTO465, ATTO655, ATTO680 and ATTO700 are all different in the observation wavelength for fluorescence quenching as described above, so that they can be used in combinations.

When the nucleic acid probe or nucleic acid probe set according to the present invention is used in the assay of one or more target nucleic acids or target nucleic acid sequences, the concentration of each target nucleic acid or target nucleic acid sequence in an assay sample may preferably be set at 1 nM to 1 μM. Further, the concentration of

each nucleic acid probe according to the present invention in the assay sample may preferably be set in a range of 0.01 to 10 moles per mole of the corresponding target nucleic acid or target nucleic acid sequence.

## Examples

Example 1 Measurement of fluorescence quenching by nucleic acid probe labeled with ATTO655

An oligonucleotide represented by SEQ ID NO: 1 was labeled with ATTO655 (product of ATTO-TEC GmbH) at a 3'-terminal cytosine thereof via a C6 linker to prepare a nucleic acid probe 1. It is to be noted that the preparation of the probe relied upon Tsukuba Oligo Service Co., Ltd. (Tsukuba, Japan). SEQ ID NO:1 5'-TTTTTTCCCCCCCCCCCC-3'

[Measurement 1]

Into a 10 mM Tris-HCl buffer (pH 8.3) containing KCl (50 mM) and $MgCl_2$ (1.5 mM), the nucleic acid probe 1 and a target nucleic acid (SEQ ID NO: 2) were added to final concentrations of 40 nM and 320 nM, respectively, to prepare a sample 1. SEQ ID NO:2 5'-GGGGGGGGGGGGAAAAAA-3'

Using a spectrophotometer ("LS50B", manufactured by PerkinElmer Co., Ltd.), the sample 1 was measured for fluorescence intensity at 630 nm to 800 nm. The measurement temperature was set at 27°C, and the slit width was set at 2 nm. The results are shown by circles (O) in FIG. 2.

[Measurement 2]

Florescence intensity was measured as in Measurement 1 except that the target nucleic acid (SEQ ID NO: 2) was not added to the sample 1. The results are shown by cross marks (X) in FIG. 2.

From the results shown in FIG. 2, it has become evident that as a result of hybridization of the nucleic acid probe 1, which is labeled with ATT0655, with the target nucleic acid (SEQ ID NO; 2), the guanine in the target nucleic acid and ATTO655 interact with each other and ATTO655 fluorescently quenches to significant extent. The extent of fluorescence quenching was pronounced on the side of wavelengths longer than 680 nm, and exceeded 90% between 682 and 800 nm. The fluorescence quenching efficiency at 684 nm was 90.9%.

Example 2 Measurement of fluorescence quenching by nucleic acid probe labeled with ATTO680

The oligonucleotide represented by SEQ ID NO: 1 was labeled with ATTO680 (product of ATTO-TEC GmbH) at the 3'-terminal cytosine thereof via a C6 linker to prepare a nucleic acid probe 2. It is to be noted that the preparation of the probe relied upon Tsukuba Oligo Service Co., Ltd. (Tsukuba, Japan).

[Measurement 3]

Into a 10 mM Tris-HCl buffer (pH 8.3) containing KCl (50 mM) and $MgCl_2$ (1.5 mM), the nucleic acid probe 2 and the target nucleic acid (SEQ ID NO:2) were added to final concentrations of 40 nM and 320 nM, respectively, to prepare a sample 2.

Using the spectrophotometer ("LS50B", manufactured by PerkinElmer Co., Ltd.), the sample 2 was measured for fluorescence intensity at 650 nm to 760 nm. The measurement temperature was set at 28°C, and the slit width was set at 2 nm. The results are shown by circles (O) in FIG. 3.

[Measurement 4]

Florescence intensity was measured as in Measurement 3 except that the target nucleic acid (SEQ ID NO: 2) was not added to the sample 2. The results are shown by cross marks (X) in FIG. 3.

From the results shown in FIG. 3, it has become evident that as a result of proximation between ATTO680 and guanine through hybridization of the nucleic acid probe 2, which is labeled with ATTO680, with the target nucleic acid (SEQ ID NO: 2), ATTO680 fluorescently quenches to significant extent. The fluorescence quenching efficiency on the side of wavelengths longer than 694 nm was 80% or higher, and the fluorescence quenching efficiency at 700 nm was 84.8%.

Example 3 Measurement of fluorescence quenching by nucleic acid probe labeled with ATTO700

The oligonucleotide represented by SEQ ID NO: 1 was labeled with ATT0700 (product of ATTO-TEC GmbH) at the 3'-terminal cytosine thereof via a C6 linker to prepare a nucleic acid probe 3. It is to be noted that the preparation of the probe relied upon Tsukuba Oligo Service Co., Ltd. (Tsukuba, Japan).

[Measurement 5]

Into a 10 mM Tris-HCl buffer (pH 8.3) containing KCl (50 mM) and $MgCl_2$ (1.5 mM), the nucleic acid probe 3 and the target nucleic acid (SEQ ID NO:2) were added to final concentrations of 40 nM and 320 nM, respectively, to prepare a sample 3.
Using the spectrophotometer ("LS50B", manufactured by PerkinElmer Co., Ltd.), the sample 3 was measured for fluorescence intensity at 670 nm to 760 nm. The measurement temperature was set at 28°C, and the slit width was set at 2 nm. The results are shown by circles (O) in FIG. 4.

[Measurement 6]

Florescence intensity was measured as in Measurement 5 except that the target nucleic acid (SEQ ID NO: 2) was not added to the sample 3. The results are shown by cross marks (X) in FIG. 4.
From the results shown in FIG. 4, it has become evident that as a result of proximation between ATT0700 and guanine through hybridization of the nucleic acid probe 3, which is labeled with ATTO700, with the target nucleic acid (SEQ ID NO: 2), ATTO700 fluorescently quenches to significant extent. The fluorescence quenching efficiency on the side of wavelengths longer than 716 nm was 60% or higher, and the fluorescence quenching efficiency at 720 nm was 68.3%.
From the above-described results of Examples 1 to 3, it has been demonstrated that ATT0655, ATTO680 and ATT700 each exhibit pronounced fluorescence quenching through an interaction with guanine in a target nucleic acid and are each useful as a fluorescence quenching dye for a fluorescence quenching probe.

Example 4 Detection of target nucleic acid by fluorescence quenching probe labeled with ATTO655

An oligonucleotide represented by SEQ ID NO: 3 was labeled with ATTO655 (product of ATTO-TEC GmbH) at a 3'-terminal cytosine thereof via a C6 linker to prepare a nucleic acid probe 4. SEQ ID NO:3 5'-CCCGTTCACCTCCACCAAGTACCTGATC-3'

[Measurement 7]

Into a 10 mM Tris-HCl buffer (pH 8.7) containing KCl (50 mM) and $MgCl_2$ (1.5 mM), the nucleic acid probe 4 and a target nucleic acid (SEQ ID NO: 4) were added to final concentrations of 50 nM and 1.6 μM, respectively, to prepare a sample 4. SEQ ID NO:4 5'-GATCAGGTACTTGGTGGAGGTGAACGGG-3'
Using a real-time PCR system ("LightCycler® 480", manufactured by Roche Applied Science, Inc.), the sample 4 was measured for fluorescence intensity at 670 nm while changing its temperature from 40°C to 95°C. The results are shown by squares (□) in FIG. 7.

[Measurement 8]

Measurement of fluorescence intensity was performed as in Measurement 7 except that the target nucleic acid was not added to the sample 4. The results are shown by filled circles (●) in FIG. 7.
From the results shown in FIG. 7, it is evident that the target nucleic acid can be detected with the nucleic acid probe 4 insofar as the condition of 75°C or lower is met.
Representing, in the equation (1), the fluorescence intensity by $F_1$ when only the nucleic acid probe existed (Measurement 8) and the fluorescence intensity by $F_2$ when the target nucleic acid also existed (Measurement 7), the fluorescence quenching efficiency was determined. The results are shown in FIG. 8. As described above, it is preferred to perform the assay of a target nucleic acid by a fluorescence quenching probe under the conditions that the fluorescence quenching efficiency is 40% or higher. It is appreciated from the results of FIG. 8 that a sample temperature of approx. 70°C or lower results in a fluorescence quenching efficiency of 40% or higher and is suited for the detection of the target nucleic acid.

[Melting Curve Analysis]

Curves of values (-dF/dT) obtained by differentiating the fluorescence intensities (F), which were obtained in Measurement 7 and Measurement 8, with respect to temperature (T) are shown in FIG. 9. The values derived from Measurement 7 are shown by squares (□), while the values derived from Measurement 8 are shown by filled circles (●). From this graph, it is possible to determine that the fluorescence intensity at the temperature (Tm) where 50% of the nucleic acid probe was in a free state was at a minimum value. Concerning Measurement 8 that the target nucleic acid did not exist in the sample, no distinct minimum is observed in fluorescence intensity. With respect to Measurement 7 that the nucleic acid existed in the sample, on the other hand, a distinct minimum in fluorescence intensity can be identified between 70°C and 80°C, and Tm was 72.3°C.

Example 5 Assay of target nucleic acid by real-time PCR

To confirm if the quantification of a target nucleic acid can be performed using a nucleic acid probe according to the present disclosure, real-time PCR was performed using the nucleic acid probe 4 to assay the target nucleic acid.
The real-time PCR measurement was performed using the real-time PCR system ("LightCycler® 480", manufactured by Roche Applied Science, Inc.). The volumes of PCR reaction mixtures were all set at 20 μL. Each PCR reaction mixture contained the nucleic acid probe 4 (final concentration: 0.2 μM) as a probe, a forward primer (SEQ ID NO: 5, final concentration: 0.3 μM), a reverse primer (SEQ ID NO: 6, final concentration: 1 μM), an ITS gene derived from a nonionic surfactant degrading bacteria, said ITS gene having been isolated by Nippon Steel Kankyo Engineering Co., Ltd., as a target nucleic acid, dNTP PLUS (product of Roche Diagnostics AG, final concentration: 0.2 mM), BSA (final concentration: 0.25 mg/mL), TITANIUM Taq DNA polymerase (predetermined amount, product of Clonetech Laboratories, Inc.), and TITANIUM Taq PCR buffer (predetermined amount, product of Clonetech Laboratories, Inc.).
SEQ ID NO:5 5'-AGTATTCTGCGCATGCGGG-3'
SEQ ID NO:6 5'-CATAACATTGCCAGCAACGCTA-3'
With respect to six samples, which contained 10 copies, $10^2$ copies, $10^3$ copies, $10^4$ copies, $10^5$ copies and $10^6$ copies of the ITS gene derived from the nonionic surfactant degrading bacteria, and, as a control, a sample which did not contain the gene, a real-time PCR assay was performed in the following cycles:

(1) Hot start: 95°C, 300 seconds, once
(2) PCR cycle: 50 cycles

(2-1) 95°C, 10 seconds thermal denaturation step
(2-2) 62°C, 20 seconds annealing step
(2-3) 72°C, 10 seconds extension step

The excitation wavelength for fluorescence was set at 610 nm, and the detection wavelength was set at 670 nm.
The resulting fluorescence intensities were introduced into the following equation (2) to determine the fluorescence quenching efficiencies with respect to the six samples that contained the ITS gene.

$$\text{Fluorescence quenching efficiency} = [1-(G_{62,n}/G_{95,n})/(G_{62}/G_{95})]\times 100 \qquad (2)$$

where,

$G_{62}$: Fluorescence intensity at the time of the annealing step in a given cycle before a pronounced change is observed in fluorescence (in the 14th cycle in this test).
$G_{95}$: Fluorescence intensity at the time of the thermal denaturation step in a given cycle before a pronounced change is observed in fluorescence (in the 14th cycle in this test).
$G_{62,n}$: Fluorescence intensity at the time of the annealing step in an nth cycle.
$G_{95,n}$: Fluorescence intensity at the time of the thermal denaturation step in the nth cycle.

Curves obtained by plotting the thus-obtained fluorescence quenching efficiencies on a graph, on which PCR cycles are plotted along the abscissa, are shown in FIG. 10. It is evident from this figure that the initial amount of the ITS gene as a target nucleic acid can be specifically determined by the fluorescence quenching efficiency. By preparing a calibration curve based on these data, the target nucleic acid can be precisely quantified.

From the results of the above experiment, it has been revealed that a target nucleic acid can be accurately quantified using a nucleic acid probe according to the present disclosure.

Example 6 SNP genotyping analysis

Using a nucleic acid probe labeled with ATTO655 (the nucleic acid probe 5) according to the present disclosure, an SNP genotyping analysis was performed on the human PPARγ gene (C/G) as a target.

Genomic DNA was extracted from buccal cells of a volunteer, and corresponding to the three genetic variants (C-allele homozygote, G-allele homozygote and CG-allele heterozygote) of the PPARγ gene, genomic DNAs were prepared, respectively. It is to be noted that the C-allele homozygote is a homozygote (w) of the wild-type gene, the G-allele homozygote is a homozygote (m) of the mutant-type gene, and the CG-allele heterozygote is a heterozygote (h) of the wild-type and mutant-type genes.

One of the above-described three kinds of genomic DNAs as templates, that is, the homozygote (w) of the wild-type gene, a forward primer (SEQ ID NO: 7, final concentration: 0.15 μM), a reverse primer (SEQ ID NO: 8, final concentration: 0.5 μM), the below-described nucleic acid probe 5 (final concentration: 0.15 μM), BSA (final concentration: 0.25 mg/mL), and LC480 Genotyping Master (predetermined amount, product of Roche Applied Science, Inc.) were added to a PCR reaction vessel, and PCR was then performed to obtain an amplification product. Between the amplification product as a target nucleic acid and the nucleic acid probe 5, a denaturation curve analysis was subsequently performed. It is to be noted that the volumes of the PCR reaction mixtures were all set at 20 μL.
SEQ ID NO:7 5'-TCCATGCTGTTATGGGTG-3'
SEQ ID NO:8 5'-ACCTTGTGATATGTTTGCAGA-3'

An oligonucleotide represented by SEQ ID NO: 9 was labeled with ATTO655 (product of ATTO-TEC GmbH) at a 3'-terminal cytosine thereof via a C6 linker to prepare a nucleic acid probe 5.
SEQ ID NO:9 5'-TGACCCAGAAAGCGATTCCTTCAC-3'

The PCRs and denaturation curve analyses were performed using the real-time PCR system ("LightCycler® 480", manufactured by Roche Applied Science, Inc.). The PCR was performed in the following cycles:

(1) Hot start: 95°C, 300 seconds, once
(2) PCR cycle: 50 cycles

(2-1) 95°C, 10 seconds thermal denaturation step
(2-2) 62°C, 20 seconds annealing step
(2-3) 72°C, 10 seconds extension step

In the above-described denaturation curve analysis, the fluorescence intensity was continuously measured at 670 nm while changing the temperature of the sample from 40°C to 95°C. Values obtained by differentiating the thus-obtained fluorescence intensities with respect to the temperature are shown by circles (O) in FIG. 11.

Further, similar assays were also performed on the homozygote of the mutant-type gene, the heterozygote, and a control sample (c) which did not contain the target nucleic acid.

From the results shown in FIG. 11, the homozygote of the wild-type gene had a Tm at 66°C, and therefore, was successfully and clearly distinguished from the homozygote of the mutant-type gene (Tm: 59°C). With respect to the heterozygote, on the other hand, two Tms were observed. From these results, it has become evident that the nucleic acid probe according to the present disclosure is useful in SNP genotyping analysis.

Example 7 Measurement of fluorescence quenching by nucleic acid probe labeled with ATTO465

The oligonucleotide represented by SEQ ID NO: 9 was labeled with ATTO465 (product of ATTO-TEC GmbH) at the 3'-terminal cytosine thereof via a C6 linker to prepare a nucleic acid probe 6. It is to be noted that the preparation of the probe relied upon Tsukuba Oligo Service Co., Ltd. (Tsukuba, Japan).

[Measurement 9]

Into a 10 mM Tris-HCl buffer (pH 8.7) containing KCl (50 mM) and $MgCl_2$ (1.5 mM), the nucleic acid probe 6 and a target nucleic acid (SEQ ID NO: 10) were added to final concentrations of 50 nM and 1.6 μM, respectively, to prepare a sample 5.
SEQ ID NO:10 5'-GTGAAGGAATCGCTTTCTGGGTCA-3'

Using the real-time PCR system ("LightCycler® 480", manufactured by Roche Applied Science, Inc.), the sample 5 was measured for fluorescence intensity at an excitation wavelength of 450 nm and a measurement wavelength

of 500 nm while changing its temperature from 40°C to 95°C. The results are shown by a solid line in FIG. 12.

[Measurement 10]

Measurement of fluorescence intensity was performed as in Measurement 9 except that the target nucleic acid was not added to the sample 5. The results are shown by a dashed line in FIG. 12. From the results shown in FIG. 12, it is evident that the target nucleic acid can be detected with the nucleic acid probe 6 labeled with ATT0465 insofar as the condition of 75°C or lower is met.

Representing, in the equation (1), the fluorescence intensity by $F_1$ when only the nucleic acid probe existed (Measurement 10) and the fluorescence intensity by $F_2$ when the target nucleic acid also existed (Measurement 9), the fluorescence quenching efficiency was determined. The results are shown in FIG. 13. As described above, it is preferred to perform the assay of a target nucleic acid by a fluorescence quenching probe under the conditions that the fluorescence quenching efficiency is 40% or higher. It is appreciated from the results of FIG. 13 that a sample temperature of approx. 72°C or lower results in a fluorescence quenching efficiency of 40% or higher and is suited for the detection of the target nucleic acid.

Example 8 SNP genotyping analysis

Using a nucleic acid probe labeled with ATTO465 according to the present invention, an SNP genotyping analysis was performed on the PPARγ gene (C/G) as a target.

By a similar procedure as in Example 6 except that the nucleic probe 6 was used in place of the nucleic acid probe 5, PCR was performed to obtain an amplification product. Between the amplification product as a target nucleic acid and the nucleic acid probe 6, a denaturation curve analysis was then performed. The fluorescence intensity was measured at an excitation wavelength of 450 nm and a measurement wavelength of 500 nm. Values obtained by differentiating the thus-obtained fluorescence intensity values with respect to the temperature are shown by cross marks (X) in FIG. 14.

Further, similar assays were also performed on the homozygote (m) of the mutant-type gene and a control sample (c) which did not contain the target nucleic acid.

From the results shown in FIG. 14, the homozygote of the wild-type gene had a Tm at 69°C, and therefore, was successfully and clearly distinguished from the homozygote of the mutant-type gene (Tm: 63°C). From these results, it has become evident that the nucleic acid probe labeled with ATTO465 according to the present invention is useful in SNP genotyping analysis.

Example 9 Nucleic acid probe set

A description will be made of illustrative quantification of the human β-actin gene by using a nucleic acid probe set according to the present invention.

First, an mRNA having the full-length sequence of the human β-actin gene was obtained from the market, and 7 kinds of PCR reaction mixtures which contained $10^2$, $10^3$, $10^4$, $10^5$, $10^6$, $10^7$ and $10^8$ copies of the mRNA were prepared. By a well-known method making use of a reverse transcriptase, cDNAs were next prepared from those samples, respectively.

As reaction mixtures for real-time PCR, seven kinds of PCR reaction mixtures, which contained the cDNAs of the human β-actin gene as obtained from the above-described samples, respectively, and a PCR reaction mixture, which did not contain any of the cDNAs, were prepared. By adding a forward primer (SEQ ID NO:11) and a reverse primer (SEQ ID NO:12) to each reaction mixture, a 262-bp nucleic acid containing a target nucleic acid sequence, which was a portion (SEQ ID NO:13) of the human β-actin gene, was amplified through a PCR reaction. Further added to each reaction mixture were a binding probe formed of a base sequence (SEQ ID NO:14) and a fluorescent probe having a base sequence (SEQ ID NO:15), formed solely of LNA, and labeled with ATTO465 at a 3'-terminal nucleotide thereof.
SEQ ID NO:11 5'-CATGTACGTTGCTATCCAGGC-3'
SEQ ID NO:12 5'-CTCCTTAATGTCACGCACGAT-3'
SEQ ID NO:13 5'-GTGAGGATCTTCATGAGGTAGTCAGTCAG-3'
SEQ ID NO:14 5'-CTGACTGACTACCTCATGAAGATCCTCACTATGAGG TGGTAGGATGGGTAGTGGT-3'
SEQ ID NO:15 5'-ACCACTACCCATCCTACCACCTCATA-ATT0465-3'

The binding probe had, on the side of a 5' -end thereof, a target nucleic acid binding region formed of a base sequence complementary to the portion (SEQ ID NO:13) of the human β-actin gene, and also had, on the side of a 3'-end thereof, a fluorescent probe binding region formed of a base sequence complementary to the fluorescent probe. Upon synthesis of the binding probe, it is preferred to phosphorylate the 3'-terminal base.

On each PCR reaction mixture, a real-time PCR measurement was performed to determine the fluorescence

quenching efficiency, and a calibration curve that indicates a relationship between the initial number of the gene in the PCR reaction mixture and the fluorescence quenching efficiency in each nth cycle was prepared. Concerning a sample the initial number of the gene in which was unknown, the initial gene number of the human β-actin gene in the sample can be determined by determining the fluorescence quenching efficiency through a similar real-time PCR measurement and applying its value to the calibration curve.

Further, the use of ATTO655, ATTO680 or ATT0700 as a fluorescence quenching dye in a fluorescent probe in place of ATTO465 makes it possible to similarly quantify the corresponding target nucleic acid by changing the fluorescence excitation wavelength and measurement wavelength.

**Legend**

| | |
|---|---|
| 1: | Base in a target nucleic acid, said base being to come to a position where it is to be paired with a cytosine base in a nucleic acid probe according to the present invention, said cytosine base being labeled with a fluorescence quenching dye |
| 1-3: | Bases in the range of 5 bases centering at the base 1 |
| 4: | Fluorescence quenching dye |
| 5: | Nucleic acid probe according to the present invention |
| 6: | Fluorescent probe |
| 7: | Fluorescent probe binding region |
| 8: | Target nucleic acid binding region |
| 9: | Binding probe |
| 10: | Target nucleic acid |
| 11: | Target nucleic acid sequence |
| 12: | One of terminal bases in the target nucleic acid binding region, said one terminal base being on the side of the fluorescence probe binding region |
| 13: | 5'-Side terminal base in the target nucleic acid binding region |
| 14: | 3'-Side terminal base in the target nucleic acid binding region |
| 41: | First fluorescence quenching dye |
| 42: | Second fluorescence quenching dye |
| 61: | First fluorescent probe |
| 62: | Second fluorescent probe |
| 71: | First fluorescent probe binding region |
| 72: | Second fluorescent probe binding region |

## Claims

1. A nucleic acid probe having a base sequence hybridizable with a target nucleic acid sequence in a target nucleic acid, wherein:

    at least one terminal base is labeled with a fluorescence quenching dye,
    the base labeled with the fluorescence quenching dye is cytosine,
    the base sequence is designed such that upon hybridization of the nucleic acid probe with the target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and
    the fluorescence quenching dye is a fluorescence quenching dye selected from the group consisting of ATT0465, ATTO680 and ATTO700.

2. A target nucleic acid sequence assaying kit comprising:

    a first nucleic acid probe having a base sequence hybridizable with a first target nucleic acid sequence, wherein:

        at least one terminal base is labeled with a fluorescence quenching dye,
        the base labeled with the fluorescence quenching dye is cytosine,
        the base sequence is designed such that upon hybridization of the nucleic acid probe with the first target nucleic acid sequence, a base in the first target nucleic acid sequence, said base being to be paired with the cytosine base, is a guanine base, or at least one guanine base exists in a range of five bases centering at the base in the first target nucleic acid sequence, said base being to be paired with the cytosine base, and
        the fluorescence quenching dye is a fluorescence quenching dye selected from the group consisting of BODIPY-FL, Pacific Blue, TAMRA and CR6G; and
        a second nucleic acid probe having a base sequence hybridizable with a second target nucleic acid sequence, wherein:

            at least one terminal base is labeled with a fluorescence quenching dye,
            the base labeled with the fluorescence quenching dye is cytosine,
            the base sequence is designed such that upon hybridization of the nucleic acid probe with the second target nucleic acid sequence, a base in the second target nucleic acid sequence, said base being to be paired with the cytosine base, is a guanine base, or at least one guanine base exists in a range of five bases centering at the base in the second target nucleic acid sequence, said base being to be paired with the cytosine base, and
            the fluorescence quenching dye is a fluorescence quenching dye selected from the group consisting of ATTO465, ATTO680 and ATTO700.

3. A method for detecting target nucleic acid sequences in a sample, comprising the following steps:

    adding a first nucleic acid probe and a second nucleic acid probe to the sample,
    measuring fluorescence derived from the first nucleic acid probe and fluorescence derived from the second nucleic acid probe while maintaining the sample under conditions where the nucleic acid probes and first and second ones of the target nucleic acid sequences are hybridizable, respectively, and
    measuring fluorescence derived from the first nucleic acid probe and fluorescence derived from the second nucleic acid probe while maintaining the sample under conditions where the nucleic acid probes and the first and second ones of the target nucleic acid sequences are not hybridizable, respectively, wherein:

the first nucleic acid probe has a base sequence hybridizable with the first target nucleic acid sequence,
at least one terminal base is labeled with a fluorescence quenching dye,
the base labeled with the fluorescence quenching dye is cytosine,
the base sequence is designed such that upon hybridization of the nucleic acid probe with the first target nucleic acid sequence, a base in the target nucleic acid sequence, said base being to be paired with the cytosine base, is a guanine base, or at least one guanine base exists in a range of five bases centering at the base in the target nucleic acid sequence, said base being to be paired with the cytosine base, and
the fluorescence quenching dye is a fluorescence quenching dye selected from the group consisting of BODIPY-FL, Pacific Blue, TAMRA and CR6G; and
the second nucleic acid probe has a base sequence hybridizable with the second target nucleic acid sequence,
at least one terminal base is labeled with a fluorescence quenching dye,
the base labeled with the fluorescence quenching dye is cytosine,
the base sequence is designed such that upon hybridization of the nucleic acid probe with the second target nucleic acid sequence, a base in the second target nucleic acid sequence, said base being to be paired with the cytosine base, is a guanine base, or at least one guanine base exists in a range of five bases centering at the base in the second target nucleic acid sequence, said base being to be paired with the cytosine base, and
the fluorescence quenching dye is a fluorescence quenching dye selected from the group consisting of ATTO465, ATTO680 and ATTO700.

**4.** A nucleic acid probe set comprising a binding probe and a fluorescent probe, wherein:

the binding probe (9) comprises an oligonucleotide, and includes a target nucleic acid binding region (8) hybridizable with a target nucleic acid sequence (11) and a fluorescent probe binding region (7) hybridizable with the fluorescent probe (6), the target nucleic acid binding region has a base sequence designed such that upon hybridization with the target nucleic acid sequence, a base in the target nucleic acid sequence, said base being to be paired with a base (12) out of the terminal bases in the target nucleic acid binding region on the side of the fluorescent probe binding region, is a guanine base, or at least one guanine base exists in a range of five bases centering at the base in the target nucleic acid sequence, said base being to be paired with the terminal base, and
the fluorescent probe (6) comprises an oligonucleotide, and is labeled with a fluorescence quenching dye (4) at a 3'-terminal base thereof when the fluorescent probe binding region is located on a side of a 3'-end of the binding probe or is labeled with the fluorescence quenching dye at a 5'-terminal base thereof when the fluorescent probe binding region is located on a side of a 5'-end of the binding probe, and the fluorescence quenching dye is selected from the group consisting of ATTO465, ATTO680 and ATTO700.

**5.** A nucleic acid probe set comprising a binding probe, a first fluorescent probe and a second fluorescent probe, wherein:

the binding probe (9) comprises an oligonucleotide, and has, from a side of a 5'-end thereof, a first fluorescent probe binding region (71) hybridizable with the first fluorescent probe (61), a target nucleic acid binding region (8) hybridizable with a target nucleic acid sequence (11), and a second fluorescent probe binding region (72) hybridizable with the second fluorescent probe (62), the target nucleic acid binding region has a base sequence designed such that upon hybridization with the target nucleic acid sequence, a base in the target nucleic acid sequence, said base being to be paired with a 5'-side terminal base (13) in the target nucleic acid binding region, is a guanine base or at least one guanine base exists in a range of five bases centering at the base in the target nucleic acid sequence, said base being to be paired with the terminal base, and a base in the target nucleic acid sequence, said base being to be paired with a 3'-side terminal base (14) in the target nucleic acid binding region, is a guanine base or at least one guanine base exists in a range of five bases centering at the base in the target nucleic acid sequence, said base being to be paired with the terminal base,
the first fluorescent probe (61) comprises an oligonucleotide, and is labeled with a first fluorescence quenching dye (41) at a 5'-terminal base thereof,
the second fluorescent probe (62) comprises an oligonucleotide, and is labeled with a second fluorescence quenching dye (42) at a 3'-terminal base thereof,
the first fluorescence quenching dye is different from the second fluorescence quenching dye,
one of the first fluorescence quenching dye and second fluorescence quenching dye is a fluorescence quenching dye selected from the group consisting of ATTO465, ATTO680 and ATTO700, and the other fluorescence quenching dye is a fluorescence quenching dye selected from the group consisting of BODIPY-FL, Pacific Blue,

TAMRA, CR6G, ATTO465, ATT0655, ATTO680 and ATTO700.

**Patentansprüche**

1. Nukleinsäure-Sonde, die eine Basensequenz aufweist, die mit einer Target-Nukleinsäuresequenz in einer Target-Nukleinsäure hybridisierbar ist, wobei:

   wenigstens eine terminale Base mit einem Fluoreszenz-Quencherfarbstoff markiert ist, die mit dem Fluoreszenz-Quencherfarbstoff markierte Base Cytosin ist,
   die Basensequenz derart gestaltet ist, dass nach der Hybridisierung der Nukleinsäure-Sonde mit der Target-Nukleinsäuresequenz, eine Base in der Target-Nukleinsäuresequenz, wobei die Base mit der Cytosinbase gepaart werden soll, eine Guaninbase ist, oder wenigstens eine Guaninbase in einem Bereich von fünf Basen vorliegt, die die Base in der Target-Nukleinsäuresequenz flankieren, wobei die Base mit der Cytosinbase gepaart werden soll, und
   der Fluoreszenz-Quencherfarbstoff ein Fluoreszenz-Quencherfarbstoff ist, ausgewählt aus der Gruppe, bestehend aus ATT0465, ATTO680 und ATTO700.

2. Untersuchungs-Kit für eine Target-Nukleinsäuresequenz, umfassend:

   eine erste Nukleinsäure-Sonde mit einer Basensequenz, die mit einer ersten Target-Nukleinsäuresequenz hybridisierbar ist, wobei:

      wenigstens eine terminale Base mit einem Fluoreszenz-Quencherfarbstoff markiert ist, die mit dem Fluoreszenz-Quencherfarbstoff markierte Base Cytosin ist,
      die Basensequenz derart gestaltet ist, dass nach der Hybridisierung der Nukleinsäure-Sonde mit der ersten Target-Nukleinsäuresequenz, eine Base in der ersten Target-Nukleinsäuresequenz, wobei die Base mit der Cytosinbase gepaart werden soll, eine Guaninbase ist, oder wenigstens eine Guaninbase in einem Bereich von fünf Basen vorliegt, die die Base in der ersten Target-Nukleinsäuresequenz flankieren, wobei die Base mit der Cytosinbase gepaart werden soll, und
      der Fluoreszenz-Quencherfarbstoff ein Fluoreszenz-Quencherfarbstoff ist, ausgewählt aus der Gruppe, bestehend aus BODIPY-FL, Pacific Blue, TAMRA und CR6G; und
      eine zweite Nukleinsäure-Sonde mit einer Basensequenz, die mit einer zweiten Target-Nukleinsäuresequenz hybridisierbar ist, wobei:

         wenigstens eine terminale Base mit einem Fluoreszenz-Quencherfarbstoff markiert ist, die mit dem Fluoreszenz-Quencherfarbstoff markierte Base Cytosin ist,
         die Basensequenz derart gestaltet ist, dass nach der Hybridisierung der Nukleinsäure-Sonde mit der zweiten Target-Nukleinsäuresequenz, eine Base in der zweiten Target-Nukleinsäuresequenz, wobei die Base mit der Cytosinbase gepaart werden soll, eine Guaninbase ist, oder wenigstens eine Guaninbase in einem Bereich von fünf Basen vorliegt, die die Base in der zweiten Target-Nukleinsäuresequenz flankieren, wobei die Base mit der Cytosinbase gepaart werden soll, und
         der Fluoreszenz-Quencherfarbstoff ein Fluoreszenz-Quencherfarbstoff ist, ausgewählt aus der Gruppe, bestehend aus ATTO465, ATTO680 und ATTO700.

3. Verfahren zur Detektion einer Target-Nukleinsäuresequenz in einer Probe, umfassend die folgenden Schritte:

   Zugeben eine ersten Nukleinsäure-Sonde und einer zweiten Nukleinsäure-Sonde zu der Probe,
   Messen der Fluoreszenz, die von der ersten Nukleinsäure-Sonde stammt und der Fluoreszenz, die von der zweiten Nukleinsäure-Sonde stammt, während die Probe unter Bedingungen gehalten wird, bei denen die Nukleinsäure-Sonden und die ersten beziehungsweise die zweiten der Target-Nukleinsäuresequenzen hybridisierbar sind, und Messen der Fluoreszenz, die von der ersten Nukleinsäure-Sonde stammt und der Fluoreszenz, die von der zweiten Nukleinsäure-Sonde stammt, während die Probe unter Bedingungen gehalten wird, bei denen die Nukleinsäure-Sonden und die ersten beziehungsweise die zweiten Target-Nukleinsäuresequenzen nicht hybridisierbar sind,
   wobei:

      die erste Nukleinsäure-Sonde eine Basensequenz aufweist, die mit der ersten Nukleinsäuresequenz hybridisierbar ist,
wenigstens eine terminale Base mit einem Fluoreszenz-Quencherfarbstoff markiert ist, die mit dem Fluoreszenz-Quencherfarbstoff markierte Base Cytosin ist,
die Basensequenz derart gestaltet ist, dass nach der Hybridisierung der Nukleinsäure-Sonde mit der ersten Target-Nukleinsäuresequenz, eine Base in der Target-Nukleinsäuresequenz, wobei die Base mit der Cytosinbase gepaart werden soll, eine Guaninbase ist, oder wenigstens eine Guaninbase in einem Bereich von fünf Basen vorliegt, die die Base in der Target-Nukleinsäuresequenz flankieren, wobei die Base mit der Cytosinbase gepaart werden soll, und
der Fluoreszenz-Quencherfarbstoff ein Fluoreszenz-Quencherfarbstoff ist, ausgewählt aus der Gruppe, bestehend aus BODIPY-FL, Pacific Blue, TAMRA und CR6G; und
die zweite Nukleinsäure-Sonde eine Basensequenz aufweist, die mit einer zweiten Target-Nukleinsäuresequenz hybridisierbar ist,
wenigstens eine terminale Base mit einem Fluoreszenz-Quencherfarbstoff markiert ist, die mit dem Fluoreszenz-Quencherfarbstoff markierte Base Cytosin ist,
die Basensequenz derart gestaltet ist, dass nach der Hybridisierung der Nukleinsäure-Sonde mit der zweiten Target-Nukleinsäuresequenz, eine Base in der zweiten Target-Nukleinsäuresequenz, wobei die Base mit der Cytosinbase gepaart werden soll, eine Guaninbase ist, oder wenigstens eine Guaninbase in einem Bereich von fünf Basen vorliegt, die die Base in der zweiten Target-Nukleinsäuresequenz flankieren, wobei die Base mit der Cytosinbase gepaart werden soll, und
der Fluoreszenz-Quencherfarbstoff ein Fluoreszenz-Quencherfarbstoff ist, ausgewählt aus der Gruppe, bestehend aus ATTO465, ATTO680 und ATT0700.

4. Set von Nukleinsäure-Sonden, umfassend eine Bindungssonde und eine Fluoreszenzsonde, wobei
die Bindungssonde (9) ein Oligonukleotid umfasst und einen Bindungsbereich für die Target-Nukleinsäure (8) einschließt, der mit der Target-Nukleinsäuresequenz (11) hybridisierbar ist, und einen Bindungsbereich für die Fluoreszenzsonde (7) einschließt, der mit der Fluoreszenzsonde (6) hybridisierbar ist, wobei
der Bindungsbereich für die Target-Nukleinsäure eine Basensequenz aufweist, die derart gestaltet ist, dass nach Hybridisierung mit der Target-Nukleinsäuresequenz, eine Base in der Target-Nukleinsäuresequenz, wobei die Base mit der einer Base (12) aus den terminalen Basen in dem Bindungsbereich für die Target-Nukleinsäure auf der Seite des Bindungsbereichs für die Fluoreszenzsonde gepaart werden soll, eine Guaninbase ist, oder wenigstens eine Guaninbase in einem Bereich von fünf Basen vorliegt, die die Base in der Target-Nukleinsäuresequenz flankieren, wobei die Base mit der terminalen Base gepaart werden soll, und
die Fluoreszenzsonde (6) ein Oligonukleotid umfasst und mit einem Fluoreszenz-Quencherfarbstoff (4) an einer 3'-terminalen Base derselben markiert ist, wenn sich der Bindungsbereich für die Fluoreszenzsonde auf der Seite des 3'-Endes der Bindungssonde befindet oder mit dem Fluoreszenz-Quencherfarbstoff an der 5'-terminalen Base desselben markiert ist, wenn sich der Bindungsbereich für die Fluoreszenzsonde auf der Seite des 5'-Endes der Bindungssonde befindet, und der Fluoreszenz-Quencherfarbstoff ausgewählt ist aus der Gruppe, bestehend aus ATTO465, ATTO680 und ATTO700.

5. Set von Nukleinsäure-Sonden, umfassend eine Bindungssonde und eine erste Fluoreszenzsonde und eine zweite Fluoreszenzsonde, wobei:

die Bindungssonde (9) ein Oligonukleotid umfasst und ausgehend von der Seite des 5'-Endes derselben einen ersten Bindungsbereich für die erste Fluoreszenzsonde (71) aufweist, die mit der ersten Fluoreszenzsonde (61) hybridisierbar ist, einen Bindungsbereich für die Target-Nukleinsäure (8), der mit der Target-Nukleinsäuresequenz (11) hybridisierbar ist, und einen zweiten Bindungsbereich für die Fluoreszenzsonde (72) aufweist, die mit der zweiten Fluoreszenzsonde (62) hybridisierbar ist, wobei der Bindungsbereich für die Target-Nukleinsäure eine Basensequenz aufweist, die derart gestaltet ist, dass nach Hybridisierung mit der Target-Nukleinsäuresequenz, eine Base in der Target-Nukleinsäuresequenz, wobei die Base mit einer terminalen Base auf der 5'-Seite (13) in dem Bindungsbereich für die Target-Nukleinsäure gepaart werden soll, eine Guaninbase ist, oder wenigstens eine Guaninbase in einem Bereich von fünf Basen vorliegt, die die Base in der Target-Nukleinsäuresequenz flankieren, wobei die Base mit der terminalen Base gepaart werden soll, und
eine Base in der Target-Nukleinsäuresequenz, wobei die Base mit einer terminalen Base auf der 3'-Seite (14) in dem Bindungsbereich für die Target-Nukleinsäure gepaart werden soll, eine Guaninbase ist, oder wenigstens eine Guaninbase in einem Bereich von fünf Basen vorliegt, die die Base in der Target-Nukleinsäuresequenz flankieren, wobei die Base mit der terminalen Base gepaart werden soll,
die erste Fluoreszenzsonde (61) ein Oligonukleotid umfasst und mit einem ersten Fluoreszenz-Quencherfarbstoff (41) an einer 5'-terminalen Base derselben markiert ist, die zweite Fluoreszenzsonde (62) ein Oligonucleotid

umfasst und mit einem zweiten Fluoreszenz-Quencherfarbstoff (42) an einer 3'-terminalen Base derselben markiert ist, sich der erste Fluoreszenz-Quencherfarbstoff von dem zweiten Fluoreszenz-Quencherfarbstoff unterscheidet,
einer von dem ersten Fluoreszenz-Quencherfarbstoff und dem zweiten Fluoreszenz-Quencherfarbstoff ein Fluoreszenz-Quencherfarbstoff ist, ausgewählt aus der Gruppe, bestehend aus ATTO465, ATTO680 und ATTO700, und der andere Fluoreszenz-Quencherfarbstoff ein Fluoreszenz-Quencherfarbstoff ist, ausgewählt aus der Gruppe, bestehend aus BODIPY-FL, Pacific Blue, TAMRA, CR6G, ATT0465, ATTO655, ATTO680 und ATT0700.

**Revendications**

1. Sonde d'acide nucléique présentant une séquence de bases pouvant s'hybrider avec une séquence d'acide nucléique cible dans un acide nucléique cible, dans laquelle :

   au moins une base terminale est marquée avec un colorant extincteur de fluorescence,
   la base marquée avec le colorant extincteur de fluorescence est la cytosine,
   la séquence de bases est conçue de sorte que, suite à l'hybridation de la sonde d'acide nucléique avec la séquence d'acide nucléique cible, une base dans la séquence d'acide nucléique cible, ladite base devant être appariée avec la base cytosine, soit une base guanine, ou au moins une base guanine existe dans une plage de cinq bases centrées sur la base dans la séquence d'acide nucléique cible, ladite base devant être appariée avec la base cytosine, et
   le colorant extincteur de fluorescence est un colorant extincteur de fluorescence choisi dans l'ensemble constitué par ATT0465, ATTO680 et ATTO700.

2. Kit de dosage de séquence d'acide nucléique cible comprenant :

   une première sonde d'acide nucléique présentant une séquence de bases pouvant s'hybrider avec une première séquence d'acide nucléique cible, dans laquelle :

      au moins une base terminale est marquée avec un colorant extincteur de fluorescence,
      la base marquée avec le colorant extincteur de fluorescence est la cytosine,
      la séquence de bases est conçue de sorte que, suite à l'hybridation de la sonde d'acide nucléique avec la première séquence d'acide nucléique cible, une base dans la première séquence d'acide nucléique cible, ladite base devant être appariée avec la base cytosine, soit une base guanine, ou au moins une base guanine existe dans une plage de cinq bases centrées sur la base dans la première séquence d'acide nucléique cible, ladite base devant être appariée avec la base cytosine, et
      le colorant extincteur de fluorescence est un colorant extincteur de fluorescence choisi dans l'ensemble constitué par BODIPY-FL, Pacific Blue, TAMRA et CR6G ; et
      une deuxième sonde d'acide nucléique présentant une séquence de bases pouvant s'hybrider avec une deuxième séquence d'acide nucléique cible, dans laquelle :

         au moins une base terminale est marquée avec un colorant extincteur de fluorescence,
         la base marquée avec le colorant extincteur de fluorescence est la cytosine,
         la séquence de bases est conçue de sorte que, suite à l'hybridation de la sonde d'acide nucléique avec la deuxième séquence d'acide nucléique cible, une base dans la deuxième séquence d'acide nucléique cible, ladite base devant être appariée avec la base cytosine, soit une base guanine, ou au moins une base guanine existe dans une plage de cinq bases centrées sur la base dans la deuxième séquence d'acide nucléique cible, ladite base devant être appariée avec la base cytosine, et
         le colorant extincteur de fluorescence est un colorant extincteur de fluorescence choisi dans l'ensemble constitué par ATT0465, ATTO680 et ATTO700.

3. Procédé pour détecter des séquences d'acides nucléiques cibles dans un échantillon, comprenant les étapes suivantes :

   addition à l'échantillon d'une première sonde d'acide nucléique et d'une deuxième sonde d'acide nucléique,
   mesure de la fluorescence dérivée de la première sonde d'acide nucléique et de la fluorescence dérivée de la deuxième sonde d'acide nucléique alors que l'échantillon est maintenu dans des conditions dans lesquelles

les sondes d'acides nucléiques et les première et deuxième des séquences d'acides nucléiques cibles peuvent s'hybrider, respectivement, et

mesure de la fluorescence dérivée de la première sonde d'acide nucléique et de la fluorescence dérivée de la deuxième sonde d'acide nucléique alors que l'échantillon est maintenu dans des conditions dans lesquelles les sondes d'acides nucléiques et les première et deuxième des séquences d'acides nucléiques cibles ne peuvent pas s'hybrider, respectivement, où :

la première sonde d'acide nucléique présente une séquence de bases pouvant s'hybrider avec la première séquence d'acide nucléique cible,
au moins une base terminale est marquée avec un colorant extincteur de fluorescence,
la base marquée avec le colorant extincteur de fluorescence est la cytosine,
la séquence de bases est conçue de sorte que, suite à l'hybridation de la sonde d'acide nucléique avec la première séquence d'acide nucléique cible, une base dans la séquence d'acide nucléique cible, ladite base devant être appariée avec la base cytosine, soit une base guanine, ou au moins une base guanine existe dans une plage de cinq bases centrées sur la base dans la séquence d'acide nucléique cible, ladite base devant être appariée avec la base cytosine, et
le colorant extincteur de fluorescence est un colorant extincteur de fluorescence choisi dans l'ensemble constitué par BODIPY-FL, Pacific Blue, TAMRA et CRGG ; et
la deuxième sonde d'acide nucléique a une séquence de bases pouvant s'hybrider avec la deuxième séquence d'acide nucléique cible,
au moins une base terminale est marquée avec un colorant extincteur de fluorescence,
la base marquée avec le colorant extincteur de fluorescence est la cytosine,
la séquence de bases est conçue de sorte que, suite à l'hybridation de la sonde d'acide nucléique avec la deuxième séquence d'acide nucléique cible, une base dans la deuxième séquence d'acide nucléique cible, ladite base devant être appariée avec la base cytosine, soit une base guanine, ou au moins une base guanine existe dans une plage de cinq bases centrées sur la base dans la deuxième séquence d'acide nucléique cible, ladite base devant être appariée avec la base cytosine, et
le colorant extincteur de fluorescence est un colorant extincteur de fluorescence choisi dans l'ensemble constitué par ATT0465, ATT0680 et ATT0700.

**4.** Jeu de sondes d'acides nucléiques comprenant une sonde de liaison et une sonde fluorescente, dans lequel :

la sonde de liaison (9) comprend un oligonucléotide, et présente une région se liant à un acide nucléique cible (8) pouvant s'hybrider avec une séquence d'acide nucléique (11) et une région se liant à une sonde fluorescente (7) pouvant s'hybrider avec la sonde fluorescente (6),
la région se liant à un acide nucléique cible présente une séquence de bases conçue de sorte que, suite à une hybridation avec la séquence d'acide nucléique cible, une base dans la séquence d'acide nucléique cible, ladite base devant être appariée avec une base (12) parmi les bases terminales dans la région se liant à un acide nucléique cible sur le côté de la région se liant à une sonde fluorescente soit une base guanine, ou au moins une base guanine existe dans une plage de cinq bases centrées sur la base dans la séquence d'acide nucléique cible, ladite base devant être appariée avec la base terminale, et
la sonde fluorescente (6) comprend un oligonucléotide, et est marquée avec un colorant extincteur de fluorescence (4) au niveau d'une base 3'-terminale de celle-ci quand la région se liant à une sonde fluorescente est située sur un côté d'une extrémité 3' de la sonde de liaison ou est marquée avec le colorant extincteur de fluorescence au niveau d'une base 5'-terminale de celle-ci quand la région se liant à une sonde fluorescente est située sur un côté d'une extrémité 5' de la sonde de liaison, et le colorant extincteur de fluorescence est choisi dans l'ensemble constitué par ATTO465, ATTO680 et Art0700.

**5.** Jeu de sondes d'acides nucléiques comprenant une sonde de liaison, une première sonde fluorescente et une deuxième sonde fluorescente, dans lequel :

la sonde de liaison (9) comprend un oligonucléotide, et présente, depuis un côté de son extrémité 5', une première région se liant à une sonde fluorescente (71) pouvant s'hybrider avec la première sonde fluorescente (61), une région se liant à un acide nucléique cible (8) pouvant s'hybrider avec une séquence d'acide nucléique cible (11), et une deuxième région se liant à une sonde fluorescente (72) pouvant s'hybrider avec la deuxième sonde fluorescente (62),
la région se liant à un acide nucléique cible présente une séquence de bases conçue de sorte que, suite à une hybridation avec la séquence d'acide nucléique cible, une base dans la séquence d'acide nucléique cible, ladite

base devant être appariée avec une base terminale sur le côté 5' (13) dans la région se liant à un acide nucléique cible, soit une base guanine, ou au moins une base guanine existe dans une plage de cinq bases centrées sur la base dans la séquence d'acide nucléique cible, ladite base devant être appariée avec la base terminale, et une base dans la séquence d'acide nucléique cible, ladite base devant être appariée avec une base terminale sur le côté 3' (14) dans la région se liant à un acide nucléique cible, soit une base guanine, ou au moins une base guanine existe dans une plage de cinq bases centrées sur la base dans la séquence d'acide nucléique cible, ladite base devant être appariée avec la base terminale,
la première sonde fluorescente (61) comprend un oligonucléotide, et est marquée avec un premier colorant extincteur de fluorescence (41) au niveau d'une base 5'-terminale de celle-ci,
la deuxième sonde fluorescente (62) comprend un oligonucléotide, et est marquée avec un deuxième colorant extincteur de fluorescence (42) au niveau d'une base 3'-terminale de celle-ci,
le premier colorant extincteur de fluorescence est différent du deuxième colorant extincteur de fluorescence,
l'un parmi le premier colorant extincteur de fluorescence et le deuxième colorant extincteur de fluorescence est un colorant extincteur de fluorescence choisi dans l'ensemble constitué par ATTO465, ATTO680 et ATTO700, et l'autre colorant extincteur de fluorescence est un colorant extincteur de fluorescence choisi dans l'ensemble constitué par BODIPY-FL, Pacific Blue, TAMRA, CR6G, ATT0465, ATTO655, ATTO680 et ATT0700.

[ FIG. 1]

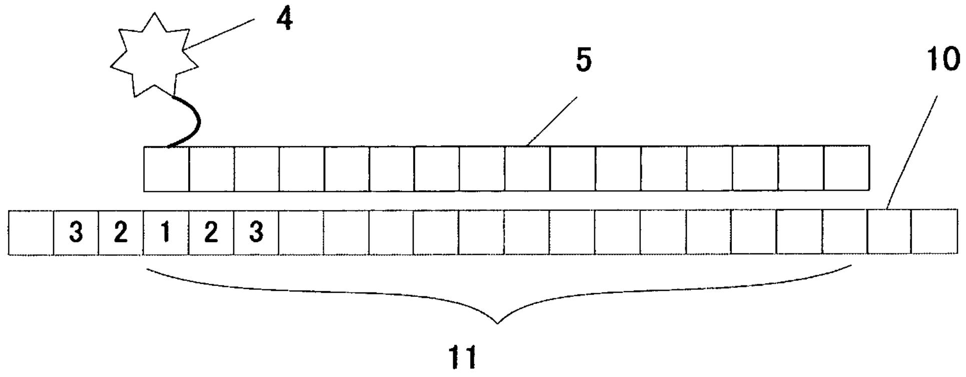
4
5
10
3
2
1
2
3
11

[ FIG. 2]

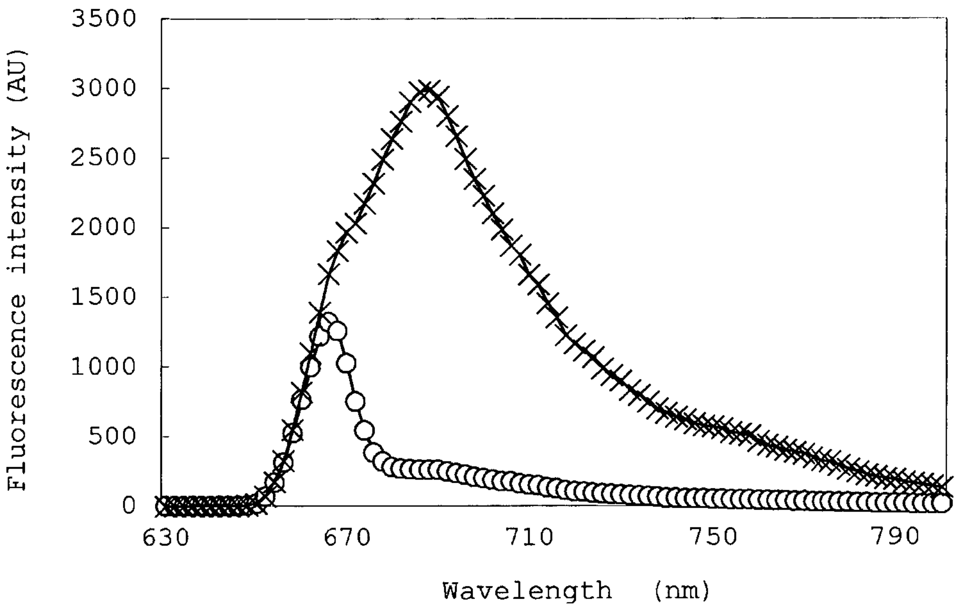
Fluorescence intensity (AU)
3500
3000
2500
2000
1500
1000
500
0
630
670
710
750
790
Wavelength (nm)

[ FIG. 3]

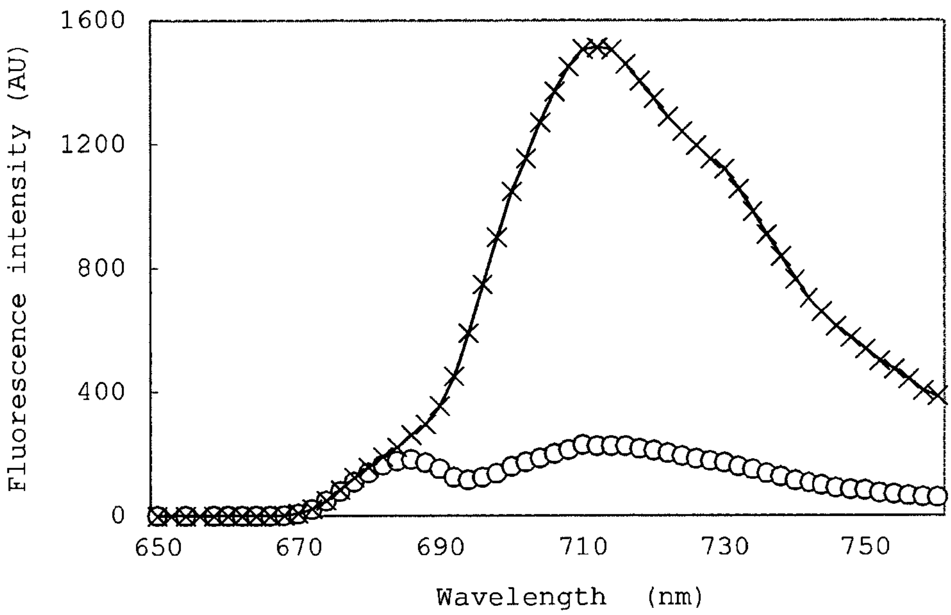
1600
1200
800
400
0
Fluorescence intensity (AU)
650
670
690
710
730
750
Wavelength (nm)

[ FIG. 4]

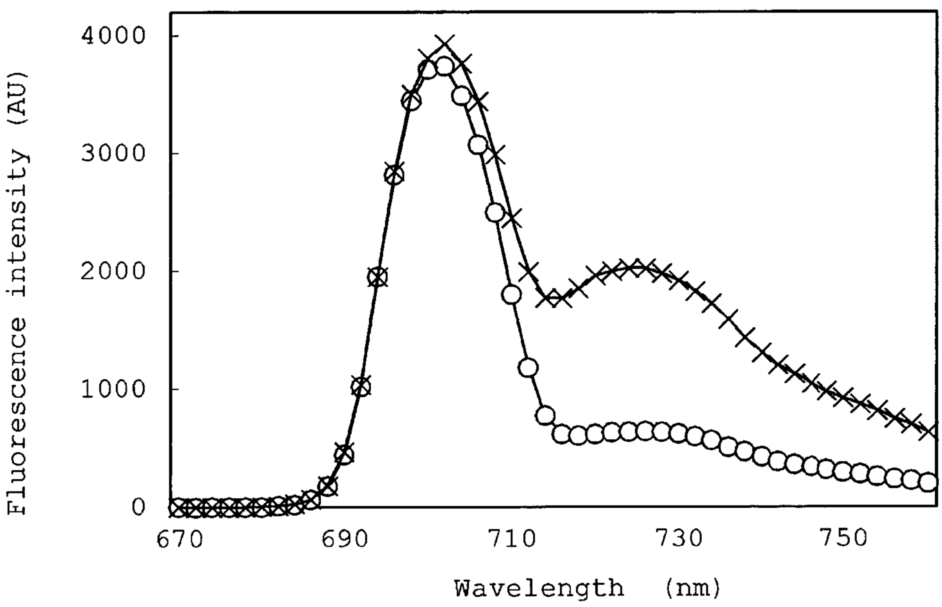
4000
3000
2000
1000
0
Fluorescence intensity (AU)
670
690
710
730
750
Wavelength (nm)

[ FIG. 5]

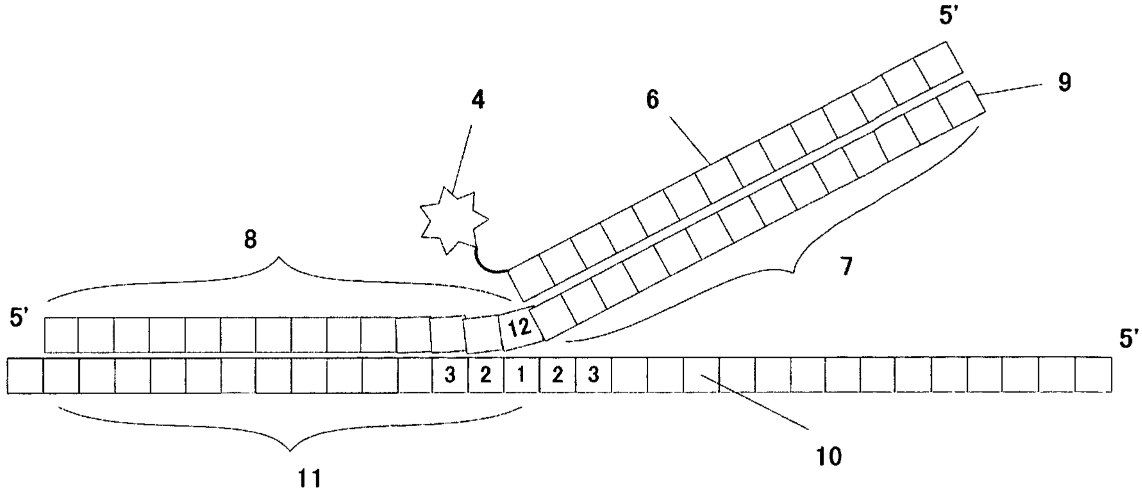
5'
4
6
9
8
7
5'
12
5'
3
2
1
2
3
10
11

[ FIG. 6]

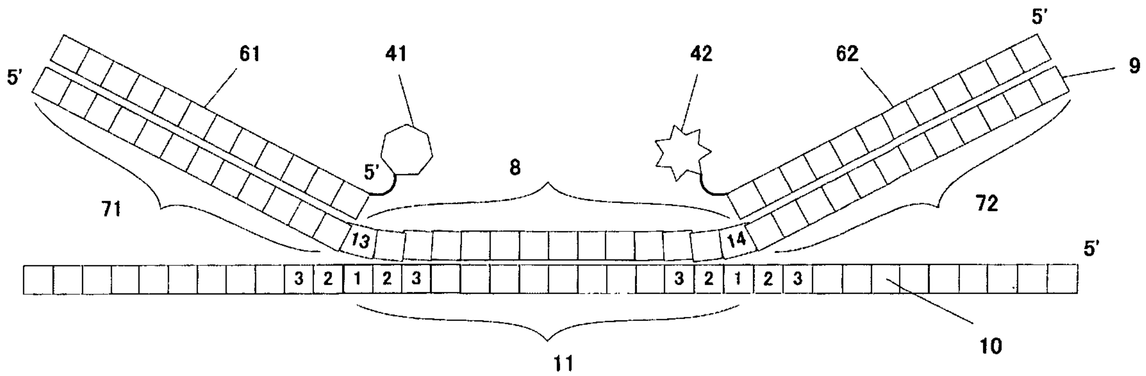
5'
61
41
42
62
9
5'
5'
8
71
72
13
14
5'
3
2
1
2
3
3
2
1
2
3
10
11

[ FIG. 7]

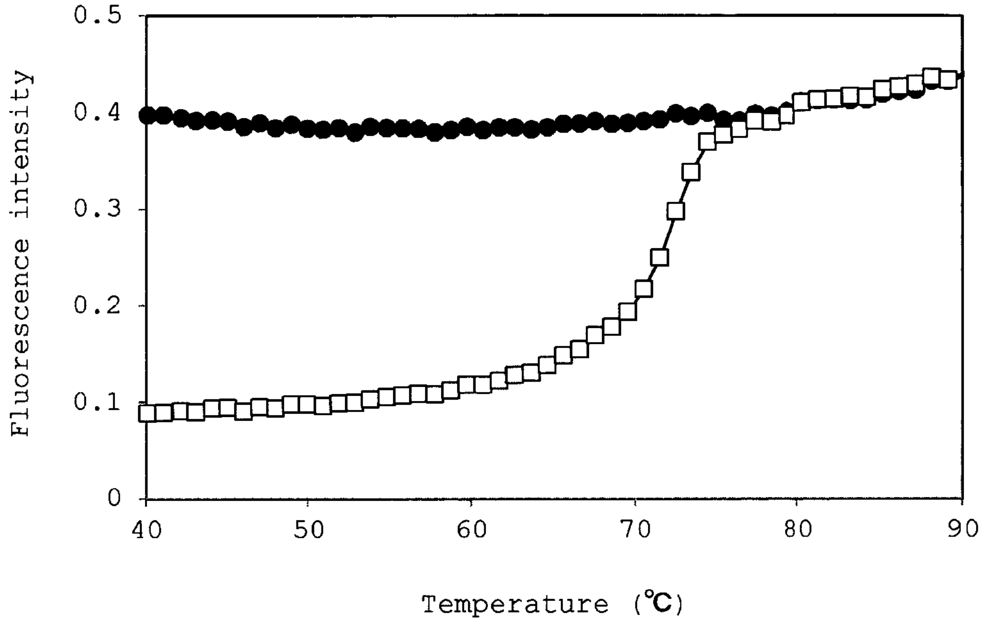
0.5
0.4
0.3
0.2
0.1
0
Fluorescence intensity
40
50
60
70
80
90
Temperature (℃)

[ FIG. 8]

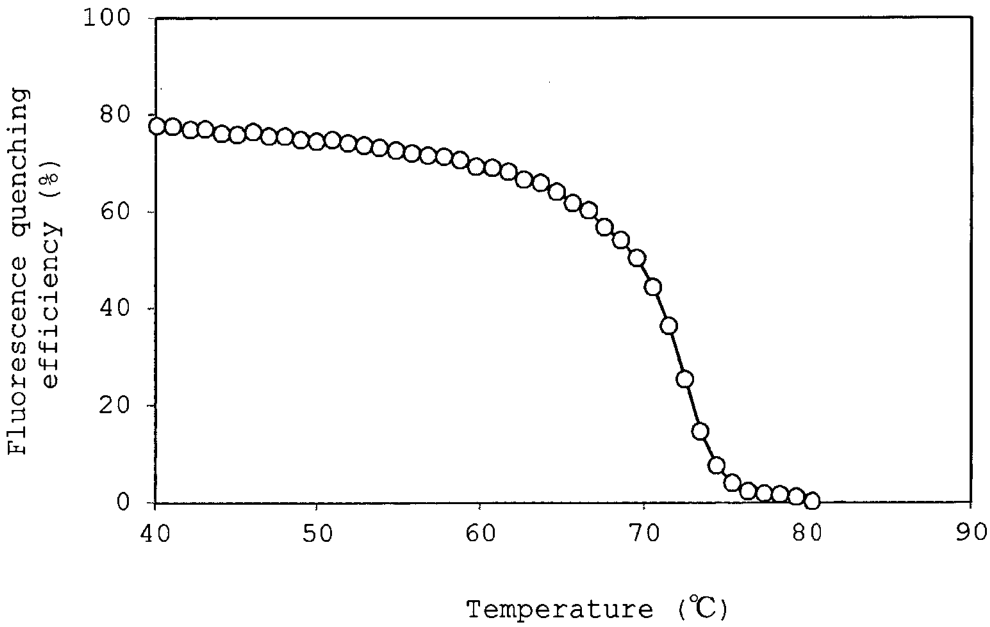
100
80
60
40
20
0
Fluorescence quenching efficiency (%)
40
50
60
70
80
90
Temperature (℃)

[ FIG. 9]

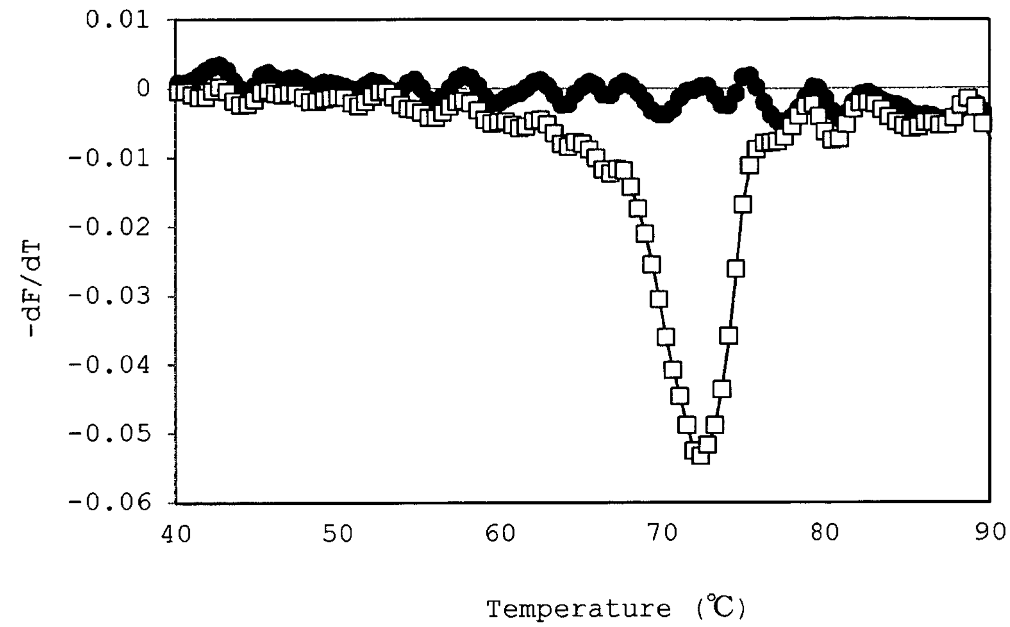
0.01
0
-0.01
-0.02
-0.03
-0.04
-0.05
-0.06
-dF/dT
40
50
60
70
80
90
Temperature (℃)

[ FIG. 10]

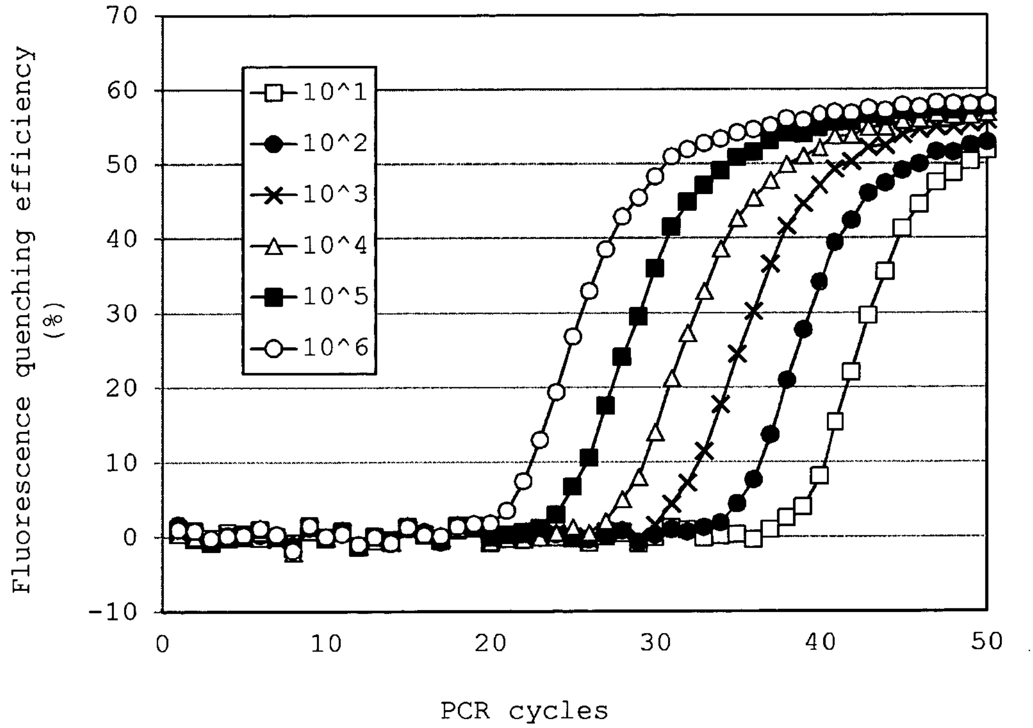
70
60
50
40
30
20
10
0
-10
Fluorescence quenching efficiency (%)
10^1
10^2
10^3
10^4
10^5
10^6
0
10
20
30
40
50
PCR cycles

[ FIG. 11]

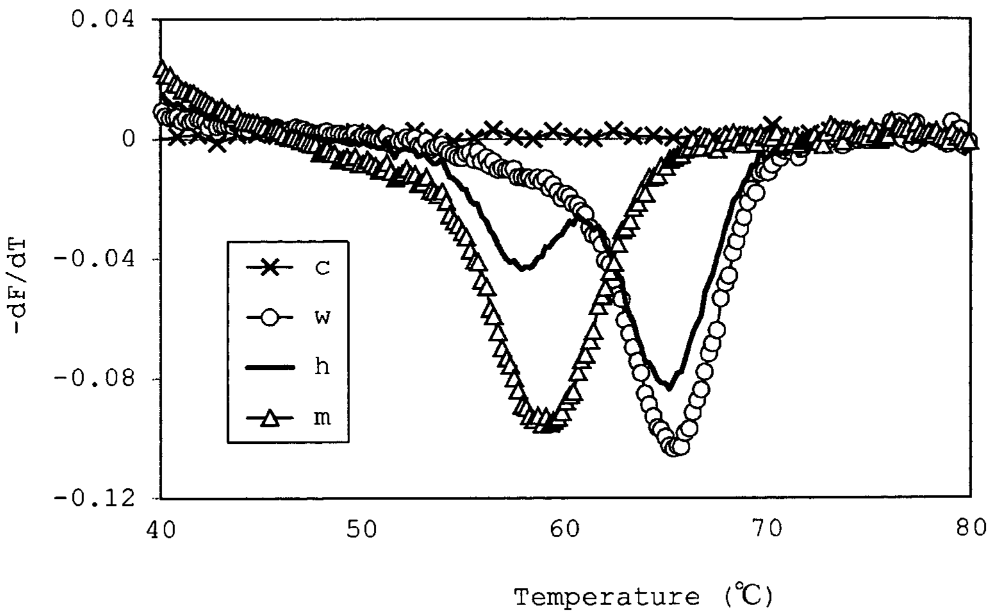
0.04
0
-0.04
-0.08
-0.12
-dF/dT
c
w
h
m
40
50
60
70
80
Temperature (℃)

[ FIG. 12]

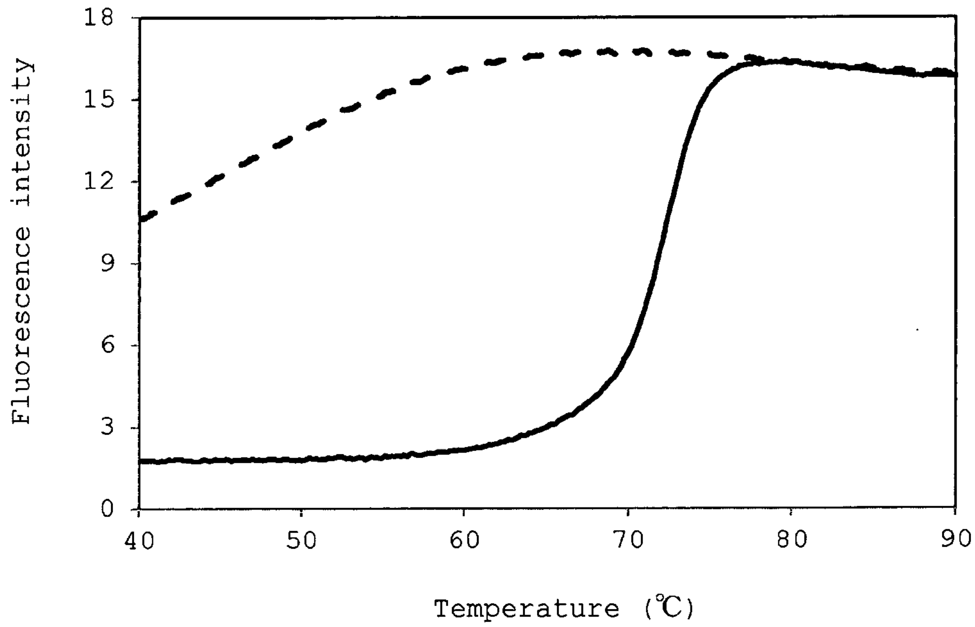
18
15
12
9
6
3
0
Fluorescence intensity
40
50
60
70
80
90
Temperature (℃)

[ FIG. 13]

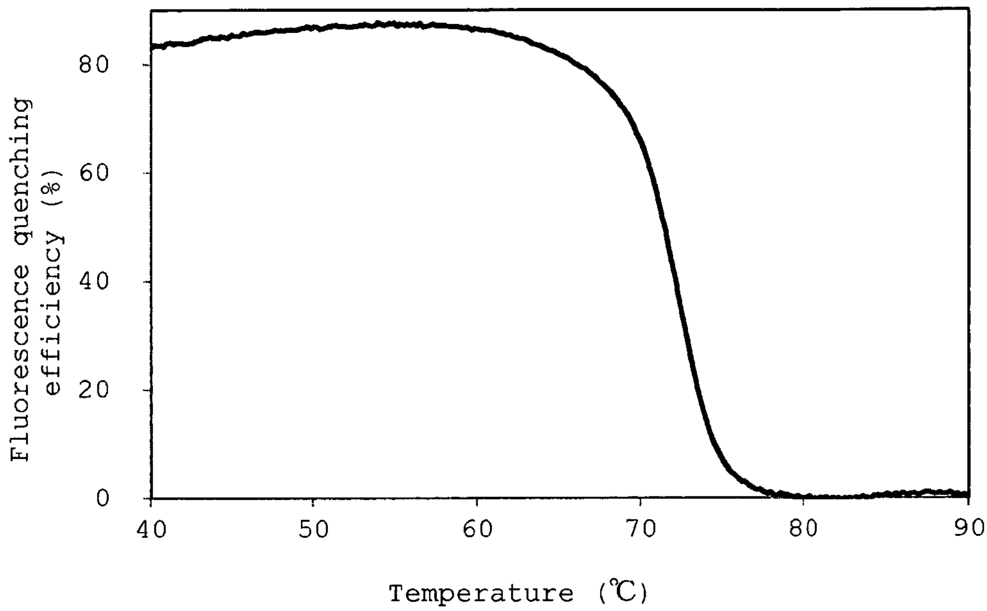
Fluorescence quenching efficiency (%)
80
60
40
20
0
40
50
60
70
80
90
Temperature (℃)

[ FIG. 14]

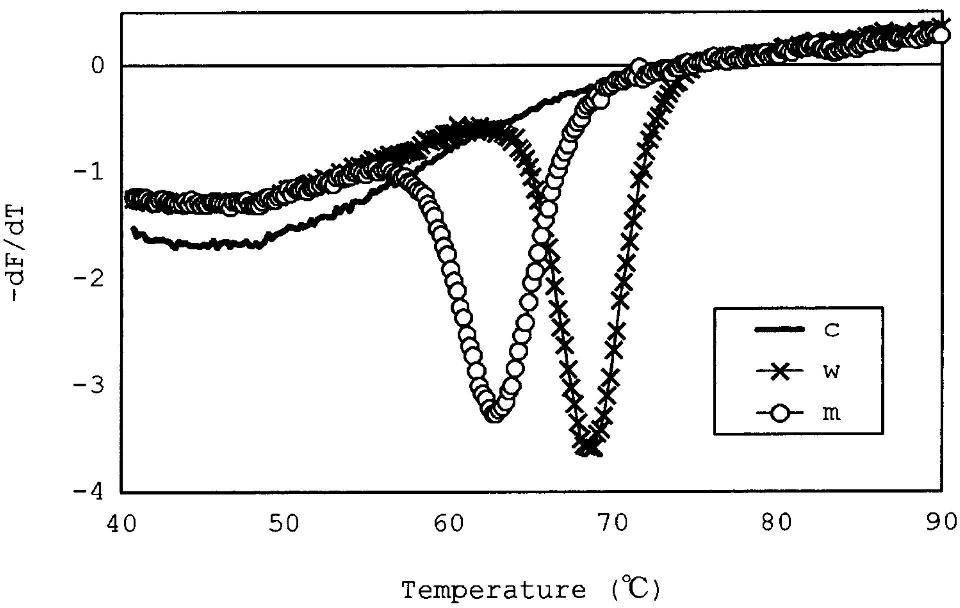
-dF/dT
0
-1
-2
-3
-4
40
50
60
70
80
90
Temperature (℃)
c
w
m

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010015719 A
- JP 3437816 B
- JP 3963422 B
- JP 2008182974 A
- WO 2010013317 A
- WO 2010013771 A
- US 20060179585 A
- JP 2011134522 A


### Non-patent literature cited in the description


- *Nucleic Acids Research,* 2001, vol. 29 (6), e34
- *Analytical Chemistry,* 15 July 2009, vol. 81 (14), 5678-5685
- *Analytical Chemistry,* 01 February 2007, vol. 79 (3), 974-979

SEQUENCE LISTING

<110> NIPPON STEEL KANKYO ENGINEERING Co., Ltd.
<120> Nucleic acid probes for measuring nucleic acids
<130> F345-P143EN
<150> JP2011-134522
<151> 2011-06-16
<160> 15

<210> 1
<211> 18
<212> DNA
<213> Artificial Sequence
<220>
<223> Nucleic Acid Probe
<400> 1
tttttcccc cccccccc 18

<210> 2
<211> 18

<212> DNA
<213> Artificial Sequence
<220>
<223> Target Nucleic Acid
<400> 2
gggggggggg ggaaaaaa 18

<210> 3
<211> 28
<212> DNA
<213> Artificial Sequence
<220>
<223> Nucleic Acid Probe
<400> 3
cccgttcacc tccaccaagt acctgatc 28

<210> 4
<211> 28
<212> DNA
<213> Artificial Sequence
<220>
<223> Target Nucleic Acid
<400> 4
gatcaggtac ttggtggagg tgaacggg 28

<210> 5
<211> 19
<212> DNA
<213> Artificial Sequence
<220>
<223> Forward Primer
<400> 5
agtattctgc gcatgcggg 19

<210> 6
<211> 22
<212> DNA
<213> Artificial Sequence
<220>
<223> Reverse Primer
<400> 6
cataacattg ccagcaacgc ta 22

<210> 7
<211> 18
<212> DNA
<213> Artificial Sequence
<220>
<223> Forward Primer
<400> 7
tccatgctgt tatgggtg 18

<210> 8
<211> 21
<212> DNA
<213> Artificial Sequence
<220>
<223> Reverse Primer

<400> 8
accttgtgat atgtttgcag a 21

<210> 9
<211> 24
<212> DNA
<213> Artificial Sequence
<220>
<223> Nucleic Acid Probe
<400> 9
tgacccagaa agcgattcct tcac 24

<210> 10
<211> 24
<212> DNA
<213> Artificial Sequence
<220>
<223> Target Nucleic Acid
<400> 10
gtgaaggaat cgctttctgg gtca 24

<210> 11
<211> 21
<212> DNA
<213> Artificial Sequence
<220>
<223> forward primer
<400> 11
catgtacgtt gctatccagg c 21

<210> 12
<211> 21
<212> DNA
<213> Artificial Sequence
<220>
<223> reverse primer
<400> 12
ctccttaatg tcacgcacga t 21

<210> 13
<211> 29
<212> DNA
<213> Homo sapiens
<400> 13
gtgaggatct tcatgaggta gtcagtcag 29

<210> 14
<211> 55
<212> DNA
<213> Artificial Sequence
<220>
<221> modified_base
<222> 55
<223> phosphorylated
<400> 14

ctgactgact acctcatgaa gatcctcact atgaggtggt aggatgggta gtggt
55

<210> 15
<400> 15
000